(19) Europäisches Patentamt
European Patent Office
Office européen des brevets

(11) EP 4 446 001 A1

(12) EUROPEAN PATENT APPLICATION

(43) Date of publication:
**16.10.2024 Bulletin 2024/42**

(21) Application number: **23167736.0**

(22) Date of filing: **13.04.2023**

(51) International Patent Classification (IPC):
***B01J 13/10*** *(2006.01)*

(52) Cooperative Patent Classification (CPC):
**B01J 13/14; A61K 8/11; A61K 8/87; A61Q 5/12; A61Q 13/00; A61Q 19/00; B01J 13/10; B01J 13/206;** A61K 2800/10; A61K 2800/412; C11D 3/505

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Givaudan SA**
**1214 Vernier (CH)**

(72) Inventor: **The designation of the inventor has not yet been filed**

(74) Representative: **Global Patents**
**Givaudan SA**
**Grafenaustrasse 7**
**6300 Zug (CH)**

# (54) IMPROVEMENTS IN OR RELATING TO ORGANIC COMPOUNDS

(57) The present invention relates to a method of forming a composition comprising at least one core-shell microcapsule, to a method for preparing the composition, the use of the composition to enhance the performance of a benefit agent in a consumer product and a consumer product comprising the composition.

EP 4 446 001 A1

## Description

### Field of the invention

The present invention relates to a composition comprising at least one benefit agent-containing core-shell microcapsule, a method of forming said composition, the use of the composition to enhance the performance of the benefit agent, and a consumer product comprising the composition.

### Background to the invention

It is known to incorporate encapsulated benefit agents in consumer products, such as household care, personal care and fabric care products. Benefit agents include for example fragrances, cosmetic agents, food ingredients, nutraceuticals, drugs and substrate enhancers.

Encapsulated benefit agents are usually presented in the form of a plurality of microcapsules. Particular examples of microcapsules are the so-called "core-shell microcapsules", typically consisting of a core containing the benefit agent encapsulated in a polymeric shell that is impervious, or at least partially impervious, to the benefit agent. Typically, these microcapsules are suspended in an aqueous medium and the encapsulated benefit agent is hydrophobic. A broad selection of materials can be used in the formation of the polymeric shell material, provided that the shell is impervious, or at least partially impervious, to the encapsulated benefit agent.

Benefit agents are encapsulated for a variety of reasons related to the improved performance of the benefit agent consumer product applications. Microcapsules can isolate and protect benefit agents from external suspending media, such as consumer product bases, in which they may be incompatible or unstable. They are also used to assist in the deposition of benefit agents onto substrates, such as skin or hair, or also fabrics or hard household surfaces in case of perfume ingredients. They can also act as a means of controlling the spatio-temporal release of a benefit agent from a consumer product.

A wide variety of encapsulating media suitable for the preparation of encapsulated compositions has been proposed in the prior art. Such encapsulating media include synthetic resins made from polyamides, polyureas, polyurethanes, polyacrylates, melamine-derived resins, or mixtures thereof.

However, consumers are increasingly concerned about using materials obtained from non-renewable sources, such as synthetic petrochemicals. In other words, consumers tend to favor materials, the origin of which is more sustainable in terms of environment and resource protection. Nevertheless, it is generally difficult to use natural materials or materials derived from nature to address all aspects of benefit agent encapsulation and performance.

In response to this, the consumer product industry has proposed microcapsules that employ more bio-sourced ingredients and which exhibit improvements in terms of biodegradability. An example of such an approach is represented by WO 2020/233887 A1, which discloses core-shell microcapsules comprising a pectin and hydoxyethylcellulose. These microcapsules are bio-sourced and show good biodegradability, while still claiming good performance in terms of stability and perfume release. However, the microencapsulation process is somewhat sensitive to the nature of the oil-phase that is to be encapsulated. This sensitivity may lead to incomplete encapsulation or the appearance of shell residues in the microcapsule slurries, as well as to the formation of microcapsules suffering from poorer storage stability due to leakage of the encapsulated core material over time.

### Summary of the invention

It is therefore a problem underlying the present invention to overcome the above-mentioned shortcomings in the prior art. In particular, a problem underlying the present invention concerns how one is to provide a robust process of preparing a core-shell microcapsule composition that is less sensitive to the nature of the oil phase that is encapsulated, whilst being formed from high levels of sustainable ingredients, in particular natural materials or materials derived from nature, that exhibit improved biodegradation, whilst keeping desirable benefit-agent release properties, during manufacture, storage and in application. Moreover, the composition should be producible in an operationally safe, robust and cost-efficient manner.

These problems are solved by the present invention, which is more fully described herein below.

Accordingly, in a first aspect, the invention provides a method of making a composition comprising a plurality of core-shell microcapsules, the method comprising the steps of

a) Providing an oil phase comprising a benefit agent to be encapsulated and a macromer formed by the reaction of one or more reactive building blocks;

b) providing an aqueous phase;

c) emulsifying the oil phase and the aqueous phase to form a dispersed phase consisting of a plurality of oil cores in a continuous aqueous phase;

d) polymerizing the macromer to form a polymeric stabilizer at the interface between the oil cores and the aqueous phase;

e) Causing a positively charged polyelectrolyte and a negatively charged polyelectrolyte, each independently added to the aqueous phase during step b), c) or d), to undergo a process of coacervation thereby to form a hydrated-polymer shell around the oil cores; and

f) Cross-linking the hydrated-polymer shell and the polymeric stabilizer in order to harden the shell around the oil cores, thereby to form a composition comprising a plurality of core-shell microcapsules.

In a second aspect the invention provides a composition comprising a plurality of core shell microcapsules obtainable by a method described in the first aspect of the invention.

In a third aspect the invention provides a consumer product comprising the composition comprising a plurality of core-shell microcapsules.

The details, embodiments, examples and preferences provided in relation to any one or more of the stated aspects of the present invention will be further described herein and apply equally to all aspects of the present invention. Any combination of embodiments, examples and preferences described herein below in all possible variations thereof are encompassed by the present invention unless otherwise indicated herein, or otherwise clearly contradicted by context.

Detailed description of the invention

The composition according to the present invention comprises a plurality of core-shell microcapsules, a microcapsule being characterized in that it consists of a core comprising a benefit agent and a polymeric shell surrounding the core. The shell is a cross-linked polymeric material formed of both the hydrated polymer and the polymeric stabilizer. A shell formed in this way provides an impervious encapsulating material around an oil core that deposits on and adheres well to substrates to which it is applied in use. Furthermore, and without being bound by any theory, it is believed that the hydrated polymer also provides an optimal point of attack for microbial degradation, thereby leading to accelerated biodegradation in nature.

The polymeric stabilizer may be in the form of a broad range of film-forming materials and resins, the precise nature of which depends upon the macromer, which in turn depends upon the selection of reactive building blocks. Preferably, the polymeric stabilizer is highly cross-linked, in order to decrease significantly the diffusion of the core contents through the shell. Preferably the imperviousness of the shell is sufficiently high to significantly prevent the leakage of the benefit agent in extractive bases, such as surfactant-containing consumer products.

In the context of the present invention, leakage may be considered to be significantly prevented if the amount of the benefit agent that has leached into a consumer product base within a period of 3 months at 40 °C is less than 75 wt.-%, preferably less than 50 wt.-%, more preferably less than 25 wt.-%, and still more preferably less than 10 wt.-% of the total amount of encapsulated benefit agent initially encapsulated.

In step a) of the method according to the invention, the macromer may be pre-formed and added to the oil phase. Alternatively, and preferably, for reasons of economy, the reactive building blocks are added to the oil phase and the macromer is formed *in-situ* in the oil phase.

The polymeric stabilizer is formed at the interface between the dispersed oil cores and the continuous aqueous phase, by polymerizing the macromer. The emulsification step c) and the step of forming the polymeric stabilizer d) may occur simultaneously or sequentially. However, without being bound by any theory, the applicant believes that the formation of the polymeric stabilizer is slower than the time required to complete the emulsification step and is completed only after step c) has been completed.

The macromer is the reaction product of the same or different reactive building blocks. The macromer may be formed by radical reaction; it may be a pre-condensate, formed by condensation; or it may be an adduct, formed by addition. Chemistries that may be suitable for the formation of macromers include but are not limited to (i) radical reaction of alpha-beta unsaturated reactive building blocks, such as (meth)acrylic acid and (meth)acrylic acid esters, styrene, divinylbenzene and vinyl esters; (ii) condensation reactions, e.g. the condensation of reactive building blocks such as carboxylic acids or carboxylic acid chlorides with alcohols or amines, the condensation of phenols or amines with aldehydes, and the condensation of silanols; (iii) addition reactions, such as the addition of reactive building blocks such as nucleophilic functional groups with activated olefinic double bonds, epoxides with alcohols, and isocyanates with alcohols or amines.

In embodiments of the invention wherein the macromer is formed *in-situ* in the oil phase, the preferred chemistries

are those set out in (i) and (iii) herein above.

As stated hereinabove, the macromer may be added, already formed, to the oil phase, or it may be formed from reactive building blocks in the oil phase. When the macromer is pre-formed, it may be prepared in an organic solvent or a water-soluble aprotic solvent.

When the macromer is formed in-situ in the oil phase, it is preferred that the oil phase is free, or substantially free, of any component that can react with the reactive building blocks and interfere with the formation of the core-shell capsules, it being understood that said component is something other than the reactive building blocks themselves.

By "substantially free" is meant that any component susceptible of reacting with the reactive building blocks is present at less than 1 wt.-%, still more particularly less than 0.1 wt.-%, based on the total weight of the oil phase.

It is also preferred that when any aforementioned component is present, it has solubility parameters such that the component is compatible with the reactive building blocks, wherein the term "compatible" has the meaning defined herein below.

Accordingly, in particular embodiments the invention is concerned with a method as defined in the first aspect of the invention wherein the macromer is formed in situ in the oil phase and wherein the oil phase is split into first and second parts such that during the formation of the macromer, the reactive building blocks are in contact with only the first part of oil phase, which is free or substantially free of any components that are reactive with the reactive building blocks.

The solubility parameters referred to above are the Hansen Solubility Parameters. Hansen Solubility Parameters are well known in the art and may be calculated by the Yamamoto-Molecular Break (Y-MB) method implemented in HSPiP software Edition 4. The person skilled in the art is familiar with using both the Hansen Solubility Parameters and the HSPiP software, including the Yamamoto-Molecular Break (Y-MB) method implemented in this software.

With regard to determining if components are compatible with the reactive building blocks based on the Solubility Parameters, it is naturally preferred that a component is compatible with all reactive building blocks. However, if different building blocks are used, and compatibility of a component is not met for all of the reactive building blocks, then it is preferred that any component contained in the oil phase is compatible with the reactive building block having the highest molecular weight.

In the context of the present invention, two substances A and B are considered to be compatible if their individual Hansen Solubility Parameters satisfy the conditions defined by Equation 1:

$$D = \sqrt{4(\delta D_A - \delta D_B)^2 + (\delta P_A - \delta P_B)^2 + (\delta H_A - \delta H_B)^2} < 9 \quad \text{(Equation 1)}$$

wherein, the parameters $\delta D$ are the dispersion parameters, the parameters $\delta P$ are the polar parameters, and the parameters $\delta H$, are the hydrogen-bonding parameters. For the sake of simplicity, the square root in Equation 1 is referred to as "D" in the specification. Substances A and B satisfying the condition $9 < D < 10$ are less compatible with each other, whereas substances A and B satisfying the conditions $D > 10$ are less compatible or even incompatible with each other.

If the reactive building blocks and the medium in which the macromer is formed are incompatible, then phase separation of the reactive building blocks and the medium may occur and the formation of the macromer may be hampered.

In embodiments of the present invention, the macromer is formed in-situ in the oil phase and the oil phase comprises, based on the total weight of the oil phase:

I. Less than 1 wt.-%, more particularly less than 0.1 wt.-%, and still more particularly 0.0 wt % of components susceptible of reacting with the reactive building blocks;

II. 40 wt.-% or more, more particularly 60 wt.-% or more, still more particularly 75 wt.-% or more still more particularly 90 wt.-% or more, still more particularly 95 wt.-% or more components having Hansen Solubility Parameters that fulfill Equation 1:

$$D = \sqrt{4(\delta D_A - \delta D_B)^2 + (\delta P_A - \delta P_B)^2 + (\delta H_A - \delta H_B)^2} < 9$$

(Equation 1)

III. Less than 60 wt.-%, more particularly less than 40 wt.-%, still more particularly less than 25 wt.-%, still more particularly less than 10 wt.-%, still more particularly less than 5 wt.-% of components having Hansen Solubility Parameters $\delta D$, $\delta P$, and $\delta H$ fulfilling the conditions given by Equation 2, and referred to as GROUP 2 components:

$$9 < \sqrt{4(\delta D_A - \delta D_B)^2 + (\delta P_A - \delta P_B)^2 + (\delta H_A - \delta H_B)^2} \leq 10$$

(Equation 2);

and

IV. less than 1 wt.-% of components, having Hansen Solubility Parameters δD, δP, and δH fulfilling the conditions given by Equation 3,

$$\sqrt{4(\delta D_A - \delta D_B)^2 + (\delta P_A - \delta P_B)^2 + (\delta H_A - \delta H_B)^2} > 10$$

(Equation 3).

wherein the subscript A refers to a particular component and subscript B refers to the reactive building block having the highest molecular weight.

In embodiments of the invention in which the macromer is formed in-situ in the oil phase, the component may be the benefit agent or it may be any part of a benefit agent when it contains multiple ingredients, such as in the case of a benefit agent being a perfume formulation comprising a plurality of perfume ingredients.

In embodiments of the present invention, when it is desired to form the macromer in-situ in the oil phase and the composition of the oil phase in total is such that it would not fulfill the compatibility conditions I) to IV) set forth above, the oil phase is split into first and second parts, such that the first part fulfills the compatibility conditions I) to IV). Said first part of the oil phase is then combined with the reactive building blocks in step a). Only after the macromer is formed is the second part of the oil phase combined with the first. In this way, components of the oil phase that could interfere with the formation of the macromer may be separated from the reactive building blocks during formation of the macromer and only combined with the first part once the macromer is formed. This can be a particularly important precaution when the oil phase comprises perfume composition that may contain multiple perfume ingredients, many of which may be reactive to some extent with the reactive building blocks.

In embodiments of the present invention in which the oil phase is split into first and second parts, the weight ratio of second to first parts is between 4.5 to 0.5 and 0.5 to 4.5, more particularly between 4 to 1 and 1 to 4, more particularly between 3 to 2 and 2 to 3. If the first part is too small compared to the second part, the amount of macromer formed may be too low to form sufficient polymeric stabilizer to adequately cover the whole oil/water surface area.

In particular embodiments of the present invention, the macromer is an adduct formed by reaction of reactive building blocks consisting of an aminosilane and a polyfunctional isocyanate. In such a case, the adduct macromer can polymerize (in step d) to form a polymeric stabilizer by a process of polycondensation at the oil/water interface. Such building blocks are described hereinafter.

In particular embodiments of the invention, the formation of the adduct macromer in step a) is performed at a temperature of from 10 °C to 50 °C, more particularly from 20 °C to 45 °C, still more particularly from 25 to 40 °C, for 15 minutes to 120 minutes, more particularly from 30 minutes to 90 minutes under stirring.

Without intending to be bound by any theory, the applicant believes that the adduct macromer is formed by addition of the amine group of the aminosilane onto an isocyanate functional group of the polyfunctional isocyanate, forming a urea bond.

Once the macromer is formed, it can polymerize to form the polymeric stabilizer at the oil/water interface by polycondensation of silane functional groups in contact with water, thereby forming siloxane bonds.

A polymeric stabilizer formed in this way is highly cross-linked and susceptible of providing reactive functional groups at its surface that can be used to immobilize additional shell-forming materials in accordance with step e) around the polymeric stabilizer thereby to complete shell formation. These reactive surface groups are isocyanate, amine and silane groups that have not reacted during the formation of the polymeric stabilizer. These additional shell-forming materials will be described in more details hereinafter, and include hydrogels, such as simple and complex coacervates.

In particular embodiments of the invention, the aqueous phase provided in step b) comprises water, more particularly deionized water, and an emulsifier, such as a surfactant, more particularly a polymeric surfactant. The term polymeric surfactant refers to a polymer that has the property of lowering the interfacial tension between an oil phase and an aqueous phase, when dissolved in one or both of the phases. The polymeric surfactant helps to promote the formation of dispersed oil droplets with desirable droplet size.

Polymeric surfactants that can be used in the process according to the invention are well known to the person skilled in the art and include a broad range of hydrocolloids, such as copolymers of acrylamide, benzene sulphonate,

(meth)acrylic acid, maleic anhydride, polyvinyl alcohol, polyvinyl pyrrolidone, and native of modified biopolymers, such as proteins, lignin and polysaccharides, and their salts.

In particular embodiments, the polymeric surfactant is a negatively charged polysaccharide.

The emulsification step c) is preferably performed at a temperature of 10 °C to 50 °C, more particularly from 20 °C to 45 °C, still more particularly from 25 to 40 °C, for 15 minutes to 120 minutes, more particularly from 30 minutes to 90 minutes.

The emulsification step c) generates a plurality of droplets that act as a template around which shell formation can occur. The droplet size distribution may be controlled by controlling the conditions of emulsification, such as stirring speed and stirrer geometry in a manner generally known in the art. As a result, a plurality of microcapsules can be obtained with controlled average size and size distribution, wherein the oil phase is encapsulated and forms thereby the core element of the core-shell microcapsules. The appropriate stirring speed and geometry of the mixer can be selected in a routine manner in order to obtain the desired average droplet size and droplet size distribution.

In particular embodiments of the invention, the emulsification step c) can be performed in a vessel equipped with a turbine, or a cross-beam stirrer with pitched beam, such as a Mig stirrer, and having a stirrer diameter to reactor diameter of 0.6 to 0.8. Microcapsules can be formed in such reactor having a volume average size (d50) of 30 μm or less, more particularly 20 μm or less, at a stirring speed from about 100 to about 1200 rpm, more particularly from about 600 to 1000 rpm. Preferably, a Mig stirrer is used operating at a speed of 850 +/-50 rpm. The person skilled in the art will however easily understand that such stirring conditions may vary depending on the size of the reactor and on the batch size, on the exact geometry of the stirrer and on the ratio of the diameter of the stirrer to the diameter of the reactor diameter ratios. For example, for a Mig stirrer with a stirrer to reactor diameter ratio from 0.5 to 0.9 and slurry volumes ranging from 0.5 to 8 tons, the preferable agitation speed in the context of the present invention is from 150 rpm to 50 rpm. It is within the purview of the skilled person to adjust the emulsification conditions having regard to the variables referred to above.

In particular embodiments of the present invention, the reactive building blocks may comprise an aminosilane. Suitable aminosilane reactive building blocks employed in the formation of the polymeric stabilizer can be selected from a compound of Formula (I).

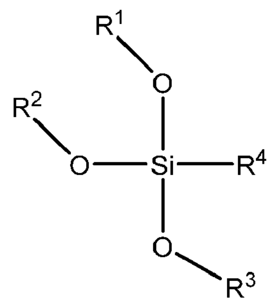

Formula (I)

In the above Formula (I), $R^1$, $R^2$ and $R^3$ are each independently $C_1$-$C_4$ linear or branched alkyl or alkenyl residues, in particular methyl or ethyl, and $R^4$ is a $C_1$-$C_{12}$, preferably a $C_1$-$C_4$, linear or branched alkyl or alkenyl residue comprising an amino functional group, in particular a primary, secondary or tertiary amine. When the functional group is a primary amine, it can be a terminal primary amine. $R^4$ is then preferably a $C_1$-$C_8$, even more preferably a $C_1$-$C_4$, linear terminal primary aminoalkyl residue. Specific aminosilanes of this category are selected from the group consisting of aminomethyltriethoxysilane, 2-aminoethyltriethoxysilane, 3-aminopropyltriethoxysilane, 4-aminobutyltri-ethoxysilane, 5-aminopentyltriethoxysilane, 6-aminohexyltriethoxysilane, 7-aminohptyltriethoxysilane and 8-aminooctyltriethoxysilane.

Without being bound by any theory, it is surmised that the silane groups polycondensate with one another to form a silica network at the oil-water interface that additionally stabilizes this interface.

In particular embodiments of the present invention, the aminosilane is a bipodal aminosilane, by which is meant a molecule comprising at least one amino group and two residues, each of these residues bearing at least one alkoxysilane moiety.

The at least one bipodal aminosilane can have the Formula (II).

$(O\text{-}R^4)_{(3\text{-}f)}(R^3)_fSi\text{-}R^2\text{-}X\text{-}R^2\text{-}Si(O\text{-}R^4)_{(3\text{-}f)}(R^3)_f$ Formula (II)

In the above Formula (II), X stands for $-NR^1-$, $-NR^1-CH_2-NR^1-$, $-NR1-CH_2-CH_2-NR^1-$, $-NR^1-CO-NR^1-$ , or

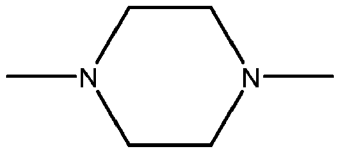

.

In the above Formula (II), $R^1$ each independently stand for H, $CH_3$ or $C_2H_5$. $R^2$ each independently stand for a linear or branched alkylene group with 1 to 6 carbon atoms. $R^3$ each independently stand for a linear or branched alkyl group with 1 to 4 carbon atoms. $R^4$ each independently stand for H or for a linear or branched alkyl group with 1 to 4 carbon atoms. f stands for 0, 1 or 2.

Bipodal aminosilanes are particularly advantageous for forming stable oil-water interfaces.

Examples of bipodal aminosilanes include, but are not limited to, bis(3-(triethoxysilyl)propyl)amine, N,N'-bis(3-(trimethoxysilyl)propyl)urea, bis(3-(methyldiethoxysilyl) propyl)amine, N,N'-bis(3-(trimethoxysilyl)propyl)ethane-1,2-diamine, bis(3-(methyldimethoxysilyl)propyl)-N-methylamine and N,N'-bis(3-(triethoxysilyl) propyl)piperazine.

The bipodal aminosilane can be a secondary bipodal aminosilane. Using a secondary bipodal aminosilane instead of primary aminosilane decreases the reactivity of the polymeric stabilizer with respect to electrophilic species, in particular aldehydes. Hence, benefit agents containing high levels of aldehydes, for example perfume ingredients containing aldehyde functionality, may be encapsulated with a lower propensity for adverse interactions between components in the oil phase and shell-forming materials.

The secondary bipodal aminosilane can be bis(3-(triethoxysilyl)propyl)amine. This particular secondary aminosilane has the advantage of releasing ethanol instead of, for instance, more toxic and less desirable methanol during the polycondensation of the ethoxysilane groups.

Other aminosilanes may also be used in combination with the aforementioned bipodal aminosilanes, in particular any of the aminosilanes described hereinabove.

In particular embodiments of the invention the reactive building blocks may comprise a polyfunctional isocyanate. Suitable polyfunctional isocyanate building blocks may be selected from organic isocyanates, in which an isocyanate group is bonded to an organic residue (R-N=C=O or R-NCO). In the context of the present invention, the polyfunctional isocyanate may be selected from alkyl, alicyclic, aromatic and alkylaromatic, as well as anionically modified polyfunctional isocyanates, with two or more (e.g. 3, 4, 5, etc.) isocyanate groups in a molecule.

Preferably, the polyfunctional isocyanate is an aromatic or an alkylaromatic isocyanate, preferably an alkylaromatic polyfunctional isocyanate having preferably methylisocyanate groups attached to an aromatic ring. Both aromatic and methylisocyanate-substituted aromatic polyfunctional isocyanates have a superior reactivity compared to alkyl and alicyclic polyfunctional isocyanates. Among these, 2-ethylpropane-1,2,3-triyl tris((3-(isocyanatomethyl)phenyl)carbamate) is particularly preferred, because of its trifunctional nature that favors the formation of intermolecular cross-links and because of its intermediate reactivity that favors network homogeneity. This alkylaromatic polyfunctional isocyanate is commercially available under the trademark Takenate D-100 N, sold by Mitsui or under the trademark Desmodur® Quix175, sold by Covestro.

As an alternative to aromatic or alkylaromatic polyfunctional isocyanates, it may also be advantageous to employ an anionically modified polyfunctional isocyanates, because of the ability of such polyfunctional isocyanates to react at the oil/water interface and even in the water phase close to the oil/water interface. A particularly suitable anionically modified polyfunctional isocyanate has the Formula (III).

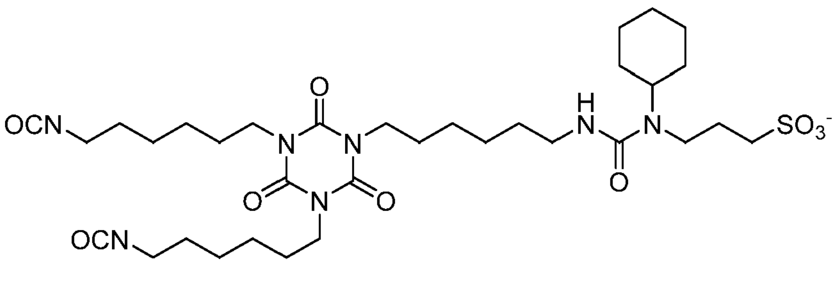

Formula (III)

Formula (III) represents a commercially available anionically modified polyisocyanate, which is a modified

isocyanate of hexamethylene diisocyanate, sold by Covestro under the trademark Bayhydur® XP2547.

In particular embodiments of the present invention step d) is carried out with a macromer that is an adduct of an aminosilane and a polyfunctional isocyanate, referred to hereinabove, and the polymeric stabilizer is formed by polycondensation of the silane groups of the adduct at the oil water interface.

Such a polycondensation may be catalyzed by either an acidic pH or an alkaline pH. For example, the pH of the aqueous phase is lower than 7, more particularly from 2.5 to 6, still more particularly from 3.5 to 5, or higher than 7, more particularly from 8 to 12, more particularly from 9 to 11. If the aqueous phase is too acidic or too alkaline, undesired hydrolysis of the siloxane bonds may occur.

The polycondensation step is advantageously performed within a temperature range of from 10 °C to 95 °C, more particularly from 25 °C to 90 °C. If the temperature is too low, the polycondensation may be too slow, whereas if the temperature is too high, the polycondensation may be too fast and uncontrollable, leading for example to the formation of microcapsule aggregates.

In particular embodiments of the invention, the step d), employing an adduct of an aminosilane and a polyfunctional isocyanate, the pH of the aqueous phase is lower than 7, more particularly from 2.5 to 6, still more particularly from 3.5 to 5; and the temperature of the emulsion is increased from from 10 °C to 95 °C, more particularly from 25 °C to 90 °C over a period of 2 hours to 6 hours, more particularly from 3 to 5 hours.

In particular embodiments of the present invention, the hydrated polymer phase in step e) is a coacervate, more particularly a complex coacervate formed from a positively charged polyelectrolyte and a negatively charged polyelectrolyte.

By the term "coacervate" as used herein is meant a polyelectrolyte-rich phase of droplets coexisting with an aqueous, polyelectrolyte poor continuous phase. The droplets can agglomerate at interfaces to form an interfacial layer. In the present context, the coacervate droplets agglomerate at the interface between the polymeric stabilizer and the aqueous phase. As a result, an encapsulated composition in water is formed, comprising a plurality of core droplets, stabilized by the polymeric stabilizer, each droplet being surrounded by coacervate droplets.

By "complex coacervation" is meant the formation of an interfacial layer comprising a mixture of polyelectrolytes.

The phenomenon of coacervation may be observed under a light microscope, wherein it is marked by the appearance of a ring around a core droplet. This ring consists of the aforementioned polyelectrolyte-rich phase that has a different refractive index than the surrounding aqueous phase.

The coacervation of a polyelectrolyte is generally induced by bringing the polyelectrolyte to its isoelectric point, meaning the point where the net charge of the polyelectrolyte is zero or close to zero. This may be achieved by changing the salt concentration or the pH of the aqueous phase. In a process of complex coacervation, complexation occurs at the pH where one of the polyelectrolytes has an overall positive electrical charge, whereas the other polyelectrolyte has an overall negative charge, so that the overall electrical charge of the complex is neutral.

As is known in the art, pH control can be used as a means of triggering coacervation. Thus, the positively charged polyelectrolyte preferably has a pH-dependent electrical charge. This is the case for polymers bearing primary, secondary and tertiary amino groups, such as polyamines, for example chitosan, and most proteins, for example gelatin. Proteins have the additional advantage of being prone to temperature-dependent structural transitions that may also be used to control the morphology of the coacervates. In particular, varying the temperature of some proteins may induce the formation of secondary, tertiary or quaternary structures of the protein that may also be used to control the properties of the coacervate.

Similarly, negatively charged polyelectrolytes bearing carboxyl groups have a pH-dependent electrical charge.

A particularly suitable coacervate is described in WO2023020883A1.

In particular embodiments of the present invention, the negatively charged polysaccharide is used as negatively charged polyelectrolyte in the formation of the complex coacervate. By using one ingredient for a dual purpose, the overall complexity and cost of the encapsulated composition can be reduced. Furthermore, the environmental impact of the product can be improved, as fewer ingredients are needed for its manufacture.

In particular embodiments of the present invention, in the step f) the complex coacervate and the polymeric stabilizer are cross-linked. Cross-linking in this manner secures the coacervate onto the polymeric stabilizer present at the core interface, providing a shell consisting of a polymer composite, rather than simply a blend of discrete polymers. Cross-linking may be achieved with a polyfunctional aldehyde, a polyfunctional epoxized compound, a polyfunctional succinimidyl compound or an enzyme, in particular transglutaminase.

Polyfunctional aldehydes may be selected from the group consisting of succinaldehyde, glutaraldehyde, glyoxal, benzene-1,2-dialdehyde, benzene-1,3-dialdehyde, benzene-1,4-dialdehyde, piperazine-N,N-dialdehyde and 2,2'-bipyridyl-5,5'-dialdehyde.

Epoxidized compounds or resins, more particularly polyfunctional glycidyl compounds, are described in co-pending application GB2203193.4. Epoxidized compounds include but are not limited to epoxidized unsaturated oils such as epoxidized soybean oil, epoxidized vegetable oil, and the like; epoxidized alcohols such as isosorbide glycidyl ether, ethylene glycol diglycidyl ether, diethylene glycol diglycidyl ether, polyethylene glycol diglycidyl ether, polypropylene glycol diglycidyl ether, glycerol polyglycidyl ether, diglycerol polyglycidyl ether, polyglycerol polyglycidyl ether, polyglycerol-3-glycidyl ether, trimethylolpropane polyglycidyl ether, neopentyl glycol diglycidyl ether, 1,6-hexanediol diglycidyl ether, pentaerythritol polyglycidyl ether; castor oil glycidyl ether; epoxidized polysaccharides such as sorbitol polyglycidyl ether; epoxidised phenols such as resorcinol diglycidyl ether, hydrogenated bisphenol A diglycidyl ether; diglycidyl terephthalate; diglycidyl o-phthalate; N-glycidyl phthalimide; epoxy cresol novolac resin; hexahydrophthalic acid diglycidyl ester; epoxidised terpenes and the like.

In the context of the present invention, a coacervate, especially a complex coacervate, which is cross-linked, in particular by covalent bonds, is considered to be a hydrogel. A *"hydrogel"* is a three-dimensional network of hydrophilic polymers that can swell in water, while maintaining its structure due to chemical or physical cross-linking of individual polymer chains.

The applicant has found that the use of hydrogels particularly enhances both the deposition and adherence of microcapsules on substrates, in particular on fabrics.

Such a hydrogel can also be formed by several methods at interfaces, especially by self-assembly of polyelectrolytes around existing interfaces, covalent grafting of pre-formed hydrogel particles in solution, polymerization of hydro-soluble monomers initiated at the interface and phase separation of water soluble macromolecules onto the interface.

The shell can additionally comprise one or more polyfunctional carboxylic acids selected from the group consisting of citric acid, benzene-1,3,5-tricarboxylic acid, benzene-1,2,4-tricarboxylic acid, 2,5-furandicarboxylic acid, itaconic acid, poly(itaconic acid) and combinations thereof. These polyfunctional carboxylic acids improve the retention of the benefit agent in the microcapsule during drying, for example once the microcapsule has been deposited on a substrate and the substrate has been dried on a line or in a dryer.

After the core-shell microcapsules are formed, the resultant composition is usually cooled to room temperature. Before, during or after cooling, the composition may be further processed. Further processing may include treatment of the composition with anti-microbial preservatives, which preservatives are well known in the art. Further processing may also include the addition of a suspending aid, such as a hydrocolloid suspending aid to assist in the stable physical dispersion of the microcapsules and prevent any creaming or coalescence. Any additional adjuvants conventional in the art may also be added during further-processing.

The method according to the present invention may comprise the additional step of drying the microcapsules, in order to render the composition in a powdered form.

Optionally, additional materials may be added to a composition in powder form, including carrier materials, such as salts, silicates, clays and carbohydrates, fire proofing materials, additional functional materials, such as fragrance ingredients, cosmetic ingredients, biologically active ingredients, and substrate enhancers, additional materials, such as polysaccharides, proteins, alkoxysilanes, synthetic polymers and copolymers, surfactants and waxes.

Drying methods such as spray-drying, spray-coating, belt and drum drying may be employed. These methods are well known to the art.

In particular, the drying process may be accompanied by an additional encapsulation process, wherein an additional functional material is entrapped in an additional encapsulating material. For example, a composition according to the invention in the form of a slurry to be dried may comprise, additionally to the core-shell microcapsules obtained in the process according to the present invention, at least one non-encapsulated functional material and at least one water-soluble encapsulating material, so that the functional material, that is not encapsulated in the core-shell microcapsule, is entrapped in the water-soluble encapsulating material during drying. Typically, the at least one water-soluble encapsulating material comprises at least one hydrocolloid, such as starch octenyl succinate and gum acacia. The hydrocolloid promotes and stabilizes the dispersion of the non-encapsulated material in the aqueous phase of the slurry, so that, upon drying, a matrix is formed around or coexisting with the core-shell microcapsules.

Encapsulated compositions described herein in dry powder form represent additional embodiments of the invention.

The functional material that is encapsulated in the core-shell microcapsules may comprise a first fragrance, whereas the functional material entrapped in the water-soluble encapsulating material may comprise a second fragrance, wherein the first and second fragrances are identical or different.

Combining at least two encapsulation processes has the advantage of providing different mechanisms for releasing the functional material, for example a combination of moisture-induced and mechanical stress-induced releases.

The drying step may also be accompanied or followed by mechanical or thermal treatment, such as spheronization, granulation and extrusion.

The second aspect of the invention provides a composition comprising a plurality of core shell microcapsules obtainable by the method described hereinabove.

In particular embodiments of the second aspect of the invention the shell of the core-shell microcapsules comprises material derived from a polymeric stabilizer that is formed by the a polycondensation of an adduct formed by

reaction of bis(3-(triethoxysilyl)propyl)amine and 2-ethylpropane-1,2,3-triyl tris((3-(isocyanatomethyl)phenyl)carbamate). Under such conditions, the oil phase or part of the oil phase in which the adduct is formed should preferably be free of amines, aldehydes and alpha-beta unsaturated alcohols. More particularly, the oil phase comprises less than 5 wt.-%, more particularly less than 1 wt.-%, still more particularly less than 0.1 wt.-% of amines, aldehydes, enols and halogenated components. Without being bound by any theory, the applicant believes that avoiding amines, aldehydes and alpha-beta unsaturated alcohols, may help mitigate undesired side reactions that can hinder the formation of core-shell microcapsules.

In these particular embodiments, the Hansen Solubility Parameters of 2-ethylpropane-1,2,3-triyl tris((3-(isocyanatomethyl)phenyl)carbamate) are used in Equations 1 to 4: $\delta D_B = 19.077$, $\delta P_B = 10.76$ and $\delta H_B = 4.81$.

In particular embodiments of the invention, the benefit agent is a fragrance composition, comprising one or more fragrance ingredients. A broad range of fragrance ingredients that can be employed in the present invention is listed herein below.

In embodiments of the invention, the fragrance ingredients have a calculated logarithm of the octanol/water partition coefficient (ClogP) that is greater than or equal to 2.8, more particularly greater than or equal to 3.2, and a molar volume greater than or equal to 150 $cm^3$/mol, more particularly greater than or equal to 170 $cm^3$/mol. Ingredients fulfilling these conditions are less prone to leaching out of the oil cores into the aqueous phase during the encapsulation process as well as after the core-shell microcapsules have been formed.

The ClogP value provides a convenient estimate of the affinity of a fragrance ingredient for the encapsulated oil phase. The higher is ClogP, the higher is the affinity of the component for the oil phase. In the context of the present invention, ClogP of an ingredient is preferably calculated by using the calculation software embedded in ChemDraw professional software version 18.1.0.535. ChemDraw is part of the ChemOffice software platform commercialized by Perkin Elmer.

The molar volume provides a convenient estimate of the tendency of an encapsulated ingredient to diffuse through the shell of a core-shell microcapsule. The higher the molar volume is, the lower is the tendency of an ingredient to diffuse through a microcapsule shell. In the context of the present invention, the molar volume may be calculated by using ACD/Labs Percepta Platform - ChemSketch Module version 2020.1.2, commercialized by ACD/Labs. In all cases, the average value is used. The person skilled in the art is familiar with using the PhysChem module of the ACD/Labs ChemSketch to calculate physicochemical properties.

As mentioned hereinabove, in particular embodiments, particularly when the macromer is to be formed in situ in the oil phase, fragrance ingredients may be split into first and second parts of the oil phase, such that the macromer is formed in the first part of the oil phase. Under such conditions, the first part of the oil phase comprises fragrance ingredients that are compatible both with the reactive building blocks forming the macromer and with the macromer itself.

In particular embodiments, the first part of the oil phase comprises, based on the total weight of the first part of the oil phase:

a) Less than 5 wt.-%, more particularly less than 1 wt.-%, still more particularly less than 0.1 wt.-% of amines, aldehydes, enols and halogenated components;

b) 40 wt.-% or more, more particularly 60 wt.-% or more, still more particularly 75 wt.-% or more still more particularly 90 wt.-% or more, still more particularly 95 wt.-% or more of components having Hansen Solubility Parameters fulfilling the conditions given by Equation 4 and referred to as GROUP 1 components:

$$D = \sqrt{4(\delta D - 19.077)^2 + (\delta P - 10.76)^2 + (\delta H - 4.81)^2} < 9$$

(Equation 4)

c) Less than 60 wt.-%, more particularly less than 40 wt.-%, still more particularly less than 25 wt.-%, still more particularly less than 10 wt.-%, sstill more particularly less than 5 wt.-% of components having Hansen Solubility Parameters fulfilling the conditions given by Equation 5, and referred to as GROUP 2 components:

$$9 < \sqrt{4(\delta D - 19.077)^2 + (\delta P - 10.76)^2 + (\delta H - 4.81)^2} \leq 10$$

(Equation 5)

d) less than 1 wt.-% of components, having Hansen Solubility Parameters given by Equation 6,

$$\sqrt{4(\delta D - 19.077)^2 + (\delta P - 10.76)^2 + (\delta H - 4.81)^2} > 10$$

(Equation 6);

e) less than 40 wt.-% of components having a ClogP from 2.5 to 3.0 and less than 5 wt.-% of components having a ClogP of less than 2.5; and

f) less than 30 wt.-% of components having a molar volume of from 150 $cm^3$/mol to 170 $cm^3$/mol and less than 5 wt.-% of components having a molar volume of less than 150 $cm^3$/mol.

Components fulfilling these conditions are less prone to leaching out of the microcapsule core into the aqueous phase during the encapsulation process and after the microcapsules have been formed.

In particular embodiments of the invention, the first part of the oil phase contains GROUP 1 components that are fragrance ingredients selected from the group consisting of (2-(1-propoxyethoxy)ethyl)benzene (Acetal R); (Z)-oxacycloheptadec-10-en-2-one (Ambrettolide); pentyl 2-phenylacetate (Amyl Phenyl Acetate); 3,5-diethyl-2,5-dimethylcyclohex-2-enone (Azarbre); 7-isopentyl-2H-benzo[b][1,4]dioxepin-3(4H)-one (Azurone); benzyl benzoate (Benzyl Benzoate); benzyl 3-phenylprop-2-enoate (Benzyl Cinnamate); benzyl 2-methylpropanoate (Benzyl Isobutyrate); benzyl 3-methylbutanoate (Benzyl Isovalerate); benzyl 2-phenylacetate (Benzyl Phenyl Acetate); benzyl 2-hydroxybenzoate (Benzyl Salicylate); 1-butoxy-1-oxopropan-2-yl butanoate (Butyl Butyro Lactate); ((1S,8aR)-1,4,4-trimethyl-2,3,3a,4,5,8-hexahydro-1H-5,8a-methanoazulen-6-yl)methanol (Cedrenol); (1S,6R,8aR)-1,4,4,6-tetramethyloctahydro-1H-5,8a-methanoazulen-6-ol (Cedrol Crystals Extra); (4Z,8Z)-1,5,9-trimethyl-13-oxabicyclo[10.1.0]trideca-4,8-diene (Cedroxyde); methyl 2-(3-oxo-2-pentylcyclopentyl)acetate (Cepionate); 3-phenylprop-2-enyl 3-phenylprop-2-enoate (Cinnamyl Cinnamate Distilled); (E)-1,1-dimethoxy-3,7-dimethylocta-2,6-diene (Citral Dimethyl Acetal); (Z)-1,1-diethoxy-3,7-dimethylocta-2,6-diene (Citrathal R); 3,7-dimethyloct-6-en-1-yl ethyl oxalate (Citronellyl Ethoxalate); 3,7-dimethyloct-6-en-1-yl formate (Citronellyl Formate); 3,7-dimethyloct-6-enenitrile (Citronellyl Nitrile); (Z)-3-methylcyclotetradec-5-enone (Cosmone); (4-methylphenyl) octanoate (Cresyl Caprylate Para); (4-methylphenyl) 2-phenylacetate (Cresyl Phenyl Acetate Para); 2-(4-methylphenoxy)acetaldehyde (Curgix); allyl 2-(cyclohexyloxy)acetate (Cyclogalbanate); 2-cyclohexylethyl acetate (Cyclohexyl Ethyl Acetate); cyclohexyl 2-hydroxybenzoate (Cyclohexyl Salicylate); 3-(4-methylcyclohex-3-en-1-yl)butan-1-ol (Cyclomethylene Citronellol); (E)-1-(2,6,6-trimethylcyclohexa-1,3-dien-1-yl)but-2-en-1-one (Damascenone); (E)-1-(2,6,6-trimethylcyclohex-2-en-1-yl)but-2-en-1-one (Damascone Alpha); (E)-1-(2,6,6-trimethyl-1-cyclohexenyl)but-2-en-1-one (Damascone Beta); 1-(2,6,6-trimethyl-1-cyclohex-3-enyl)but-2-en-1-one (Damascone Delta); decanenitrile (Decanonitrile); (oxybis(methylene))dibenzene (Dibenzyl Ether); 3-methyl-2-pentylcyclopent-2-enone (Dihydro Jasmone); 6-heptyltetrahydro-2H-pyran-2-one (Dodecalactone Delta); 5-octyloxolan-2-one (Dodecalactone Gamma); 1,4-dioxacycloheptadecane-5,17-dione (Ethylene Brassylate); 3-(4-ethylphenyl)-2,2-dimethylpropanenitrile (Fleuranil); (E)-undec-9-enenitrile (Floridile); (3aR,6S,7aS)-3a,4,5,6,7,7a-hexahydro-1H-4,7-methanoinden-6-yl propanoate (Florocyclene); 2,4,6-trimethyl-4-phenyl-1,3-dioxane (Floropal); (3aS,4S, 7R, 7aS)-ethyl octahydro-1H-4,7-methanoindene-3a-carboxylate (Fruitate); 2-methyldecanenitrile (Frutonile); 1-(1,2,8,8-tetramethyl-1,2,3,4,5,6,7,8-octahydronaphthalen-2-yl)ethanone (Georgywood); (E)-6,10-dimethylundeca-5,9-dien-2-one (Geranyl Acetone); (E)-oxacyclohexadec-12-en-2-one (Habanolide); methyl 3-oxo-2-pentylcyclopentaneacetate (Hedione); heptan-2-one (Heptone); (Z)-hex-3-en-1-yl benzoate (Hexenyl-3-Cis Benzoate); (Z)-hex-3-en-1-yl (Z)-hex-3-enoate (Hexenyl-3-Cis Hexenoate); (Z)-hex-3-en-1-yl 2-hydroxybenzoate (Hexenyl-3-Cis Salicylate); (Z)-hex-3-enyl] (E)-2-methylbut-2-enoate (Hexenyl-3-Cis Tiglate); hexyl 2-hydroxybenzoate (Hexyl Salicylate); (E)-4-(2,6,6-trimethylcyclohex-1-en-1-yl)but-3-en-2-one (Ionone Beta); (E)-3-methyl-4-(2,6,6-trimethylcyclohex-2-en-1-yl)but-3-en-2-one (Irisantheme); (E)-4-(2,6,6-trimethylcyclohex-2-en-1-yl)but-3-en-2-one (Irisone Alpha); 1-(2,3,8,8-tetramethyl-1,2,3,4,5,6,7,8-octahydronaphthalen-2-yl)ethanone (Iso E Super); 2-methylpropyl 2-phenylacetate (Isobutyl Phenyl Acetate); 2-methylpropyl 2-hydroxybenzoate (Isobutyl Salicylate); 2-hexylcyclopent-2-en-1-one (Isojasmone B 11); 2-hexylcyclopent-2-enone (Isojasmone T); (E)-3-methyl-4-(2,6,6-trimethylcyclohex-2-en-1-yl)but-3-en-2-one (Isoraldeine 70); (3aR,6S,7aS)-3a,4,5,6,7,7a-hexahydro-1H-4,7-methanoinden-6-yl acetate (Jasmacyclene); 2-hexylcyclopentanone (Jasmatone); (E)-6-(pent-3-en-1-yl)tetrahydro-2H-pyran-2-one (Jasmolactone); 3-butyl-5-methyltetrahydro-2H-pyran-4-yl acetate (Jasmonyl); 3-pentyltetrahydro-2H-pyran-4-yl acetate (Jasmopyrane); (4Z)-hept-4-en-2-yl 2-hydroxybenzoate (Karmaflor); (2E,6Z)-3,7-dimethylnona-2,6-dienenitrile (Lemonile); 3-methyl-5-phenylpentan-1-ol (Mefrosol); 5-methyl-2-propan-2-ylcyclohexyl] 2-hydroxypropanoate (Menthyl Lactate); methyl non-2-ynoate (Methyl Octyne Carbonate); (E)-3-methyl-4-(2,6,6-trimethylcyclohex-1-en-1-yl)but-3-en-2-one (Methylionantheme); (Z)-3-methylcyclopentadec-5-enone (Muscenone); 1,4-dioxacyclohexadecane-5,16-dione (Musk C14); 1,7-dioxacycloheptadecan-8-one (Musk R1); (4-(4-methylpent-3-en-1-yl)cyclohex-3-en-1-yl)methyl acetate (Myraldyl Acetate); 2-(2-(4-methylcyclohex-3-en-1-yl)propyl)cyclopentan-1-one (Nectaryl); (E)-methyl non-2-enoate (Neofolione); (E)-13-methyloxacyclopentadec-10-en-2-one (Nirvanolide); 4,4a-dimethyl-6-(prop-1-en-2-yl)-4,4a,5,6,7,8-hexahydronaphthalen-2(3H)-one (Nootkatone Crystals); 4-(tert-pentyl)cyclohexanone

(Orivone); 2-ethyl-N-methyl-N-(m-tolyl)butanamide (Paradisamide); 1,1-dimethoxynon-2-yne (Parmavert); 5-heptyldihydrofuran-2(3H)-one (Peach Pure); 2-cyclohexylidene-2-phenylacetonitrile (Peonile); 3,7-dimethylocta-1,6-dien-3-yl dimethylcarbamate (Pepperwood); 2-cyclohexylidene-2-(o-tolyl)acetonitrile (Petalia); 2-(phenoxy)ethyl 2-methylpropanoate (Phenoxy Ethyl Isobutyrate); 2-phenylethyl 3-phenylprop-2-enoate (Phenyl Ethyl Cinnamate); 2-phenylethyl 2-methylpropanoate (Phenyl Ethyl Isobutyrate); 2-phenylethyl 2-phenylacetate (Phenyl Ethyl Phenyl Acetate); 2-phenylethyl 2-hydroxybenzoate (Phenyl Ethyl Salicylate Crystals); (2E,5E)-5,6,7-trimethylocta-2,5-dien-4-one (Pomarose); (4aR,8aS,E)-6-ethylideneoctahydro-2H-5,8-methanochromene (Rhuboflor); 3-(2-methylpropyl)-1-methylcyclohexanol (Rossitol); 2,3,3-trimethyl-1-indanone (Safraleine); 4,5,6,7,8,9,10,11,12,13-decahydrocyclododeca[d]oxazole (Sclarene 50%/Tec); 1-(spiro[4.5]dec-6-en-7-yl)pent-4-en-1-one (Spirogalbanone Pure); (E)-tridec-2-enenitrile (Tridecene-2-Nitrile); 6-hexyltetrahydro-2H-pyran-2-one (Undecalactone Delta); (Z)-cyclohexadec-5-enone (Velvione); or mixtures thereof.

In particular embodiments of the invention, the first part of the oil phase contains GROUP 2 components that are fragrance ingredients selected from the group consisting of octyl acetate (Acetate C 8 Octylic); (Z)-4,11,11-trimethyl-8-methylenebicyclo[7.2.0]undec-4-ene (Acetyl Caryophyllene); 2-methyl-4-(5,6,6-trimethylbicyclo[2.2.1]hept-2-yl-cyclohexanone (Aldrone); prop-2-enyl 2-(3-methylbutoxy)acetate (Allyl Amyl Glycolate); prop-2-enyl heptanoate (Allyl Oenanthate); 1-((2-(tert-butyl)cyclohexyl)oxy)butan-2-ol (Amber Core); 1,3,4,5,6,7-hexahydro-.beta.,1,1,5,5-pentamethyl-2H-2,4a-Methanonaphthalene-8-ethanol (Ambermax); (2-(isopentyloxy)ethyl)benzene (Anther); 1-(3,3-dimethylcyclohexyl)ethyl formate (Aphermate); (E)-2-ethyl-4-(2,2,3-trimethylcyclopent-3-en-1-yl)but-2-en-1-ol (Bacdanol); (1R,2S,4R)-2'-isopropyl-1,7,7-trimethylspiro[bicyclo[2.2.1]heptane-2,4'-[1,3]dioxane] (Belambre); 2-methyl-6-methyleneoct-7-en-2-yl acetate (Bergamyl Acetate); (ethoxymethoxy)cyclododecane (Boisambrene Forte); (2S,4S)-1,7,7-trimethylbicyclo[2.2.1]heptan-2-yl acetate (Bornyl Acetate); 4-(tert-butyl)cyclohexanol (Butyl Cyclohexanol Para); 1,1,2,3,3-pentamethyl-2,3,6,7-tetrahydro-1H-inden-4(5H)-one (Cashmeran); ((3R,3aS,7R,8aS)-3,8,8-trimethyl-2,3,4,7,8,8a-hexahydro-1H-3a,7-methanoazulen-6-yl)methyl acetate (Cedrenyl Acetate); (1S,6R,8aR)-1,4,4,6-tetramethyloctahydro-1H-5,8a-methanoazulen-6-yl acetate (Cedryl Acetate Crystals); 2-methyl-4-(2,6,6-trimethylcyclohex-2-en-1-yl)butanal (Cetonal); 3,7-dimethyloct-6-en-1-ol (Citronellol 750); 3,7-dimethyloct-6-en-1-ol (Citronellol); 3,7-dimethyloct-6-en-1-yl acetate (Citronellyl Acetate); 3,7-dimethyloct-6-en-1-yl propanoate (Citronellyl Propionate; 4-cyclohexyl-2-methylbutan-2-ol (Coranol); ethyl 2,6,6-trimethylcyclohexa-1,3-diene-1-carboxylate (Cristalon); 3,7-dimethyloct-6-en-3-ol (Dihydro Linalool); 2,6-dimethyloct-7-en-2-ol (Dihydro Myrcenol); 2-methyl-1-phenylpropan-2-yl butanoate (Dimethyl Benzyl Carbinyl Butyrate); (E)-3-methyl-5-(2,2,3-trimethylcyclopent-3-en-1-yl)pent-4-en-2-ol (Ebanol); (2E,4Z)-ethyl deca-2,4-dienoate (Ethyl Decadienoate); ethyl decanoate (Ethyl Decanoate); (E)-3,7-dimethylnona-1,6-dien-3-ol (Ethyl Linalool); ethyl octanoate; ethyl nonanoate; ethyl 2,6,6-trimethylcyclohexa-1,3-diene-1-carboxylate (Ethyl Safranate); (2S)-1,3,3-trimethylbicyclo[2.2.1]heptan-2-yl acetate (Fenchyl Acetate); 1-(3,5,5,6,8,8-hexamethyl-5,6,7,8-tetrahydronaphthalen-2-yl)ethanone (Fixolide); 2,6-dimethyloct-7-en-2-ol (Floralym); (Z)-1-(cyclooct-3-en-1-yl)propan-1-ol (Florymoss); 4,4,8,8-tetramethyloctahydro-4a,7-methanonaphtho[1,8a-b]oxirene (Folenox); (3aR,6S,7aS)-3a,4,5,6,7,7a-hexahydro-1H-4,7-methanoinden-6-yl 2-methyl propanoate (Gardocyclene); (E)-3,7-dimethylocta-2,6-dien-1-yl acetate (Geranyl Acetate Synthetic); 2-(8-isopropyl-6-methylbicyclo[2.2.2]oct-5-en-2-yl)-1,3-dioxolane (Glycolierral); (Z)-hex-3-en-1-yl butanoate; (Z)-hex-3-en-1-yl 2-methylpropanoate; hexyl butanoate; hexyl 2-methylpropanoate; (E)-4-(2,5,6,6-tetramethyl-1-cyclohex-2-enyl)but-3-en-2-one (Ironal); (E)-4-(2,5,6,6-tetramethylcyclohex-2-en-1-yl)but-3-en-2-one (Irone Alpha); 2,2,7,7-tetramethyltricyclo[6.2.1.01,6]undecan-5-one (Isolongifolanone); (1-methyl-2-((1,2,2-trimethylbicyclo[3.1.0]hexan-3-yl)methyl)cyclopropyl)methanol (Javanol); 1-(4-methoxy-2,2,6,6-tetramethylcyclohex-3-en-1-yl)ethanone (Kefarene); (Z)-1-(1-ethoxyethoxy)hex-3-ene (Leaf Acetal); 3,7-dimethylocta-1,6-dien-3-ol (Linalool Synthetic); 3,7-dimethylocta-1,6-dien-3-yl formate (Linalyl Formate); 2-(4-methylcyclohexyl)propan-2-yl acetate (Menthanyl Acetate); 2-isopropyl-5-methylcyclohexanol (Menthol); 1-((1S,8aS)-1,4,4,6-tetramethyl-2,3,3a,4,5,8-hexahydro-1H-5,8a-methanoazulen-7-yl)ethanone (Methyl Cedryl Ketone); (2-methyl-6-methylideneoct-7-en-2-yl) acetate (Myrcenyl Acetate); 2-methylundecanoic acid (Mystikal); 2-methyl-6-methyleneoct-7-en-2-yl acetate (Neobergamate Forte); 10-isopropyl-2,7-dimethyl-1-oxaspiro[4.5]deca-3,6-diene (Neocaspirene); (E)-3,7,11-trimethyldodeca-1,6,10-trien-3-ol (Nerolidol); (Z)-3,7-dimethylocta-2,6-dien-1-yl acetate (Neryl Acetate); 2-(6,6-dimethylbicyclo[3.1.1]hept-2-en-2-yl)ethyl acetate (Nopyl Acetate); 2,4-dimethyl-2-(5,5,8,8-tetramethyl-5,6,7,8-tetrahydronaphthalen-2-yl)-1,3-dioxolane (Okoumal); (1-methyl-2-(((1R,3R)-2,2,3-trimethylcyclopentyl)methyl)cyclopropyl)methanol (Pashminol); 2,2-dimethyl-2-pheylethyl propanoate (Pivarose); ethyl 2-cyclohexylpropanoate (Poirenate); (E)-2-ethyl-4-(2,2,3-trimethylcyclopent-3-en-1-yl)but-2-en-1-ol (Radjanol); 2-(cyclohexylmethyl)-4,4,6-trimethyl-1,3-dioxane (Resedal); mixture of 3,7-dimethyloct-6-en-1-ol and 3,7-dimethyloct-7-en-1-ol (Rhodinol); dec-9-en-1-ol (Rosalva); 3-((1R,2S,4R,6R)-5,5,6-trimethylbicyclo[2.2.1]heptan-2-yl)cyclohexanol (Sandela); (E)-2-methyl-4-(2,2,3-trimethyl-1-cyclopent-3-enyl)but-2-en-1-ol (Santacore); 2-(1-(3,3-dimethylcyclohexyl)ethoxy)-2-methylpropyl cyclopropanecarboxylate (Serenolide); (E)-6-ethyl-3-methyloct-6-en-1-ol (Super Muguet); (E)-2-((3,5-dimethylhex-3-en-2-yl)oxy)-2-methylpropyl cyclopropanecarboxylate (Sylkolide); (E)-6,10-dimethylundeca-5,9-dien-2-yl acetate (Tangerinol); 2-(4-methyl-1-cyclohex-3-enyl)propan-2-yl acetate (Terpinyl Acetate); oxacyclohexadecan-2-one (Thibetolide); (E)-4-methyldec-3-en-5-ol (Undecavertol); 2,2,5-trimethyl-5-pentylcyclopentanone (Veloutone); 1-((1S,8aS)-1,4,4,6-tetramethyl-2,3,3a,4,5,8-hexahydro-1H-5,8a-methanoazulen-7-yl)ethanone (Vertofix Coeur); 4-methyl-4-phenylpentan-2-yl acetate (Vetikol Acetate/Corps Rhubarb); (4,8-dimethyl-2-propan-2-ylidene-3,3a,4,5,6,8a-hexahydro-1H-azulen-6-yl) acetate (Vetiveryl Acetate); (2R,5R,8S)-4,4,8-trimethyltricyclo[6.3.1.02,5]dodecan-1-yl acetate (Vetynal); [(3Z)-4,11,11-trimethyl-8-methylidene-5-bicyclo[7.2.0]undec-3-enyl] acetate (Vetyvenal); or mixtures thereof.

Once the adduct has been formed and before the polymeric stabilizer is formed by polycondensation of the adduct at the oil-water interface has been completed, a second part of the oil phase may be added. The selection criteria of the second part of the oil phase fragrance ingredients are less restrictive in terms of compatibility with the reactive blocks and with the adduct. However, the adduct should preferably be compatible with the oil phase as a whole, so that the polycondensation of the adduct at the oil-water interface occurs. Thus, the second oil phase is preferably substantially free of incompatible fragrance ingredients, such as alpha-beta unsaturated aldehydes that otherwise could react with residual unreacted amino groups remaining in the adduct. By "substantially free" is meant that the incompatible ingredients such as the alpha-beta unsaturated aldehydes should be present at less than 5 wt.-%, more particularly less than 1 wt.-%, still more particularly less than 0.1 wt.-%, based on the total weight of the second oil phase.

In preferred embodiments of the invention in which the oil phase is split into first and second parts, and the first part is as hereinabove defined, the second part of the oil phase comprises, based on the total weight of the second oil phase:

a) Less than 5 wt.-%, more particularly less than 1 wt.-%, still more particularly less than 0.1 wt.-% of amines and alpha-beta unsaturated aldehydes;

b) 15 wt.-% or less of aldehydes that are not alpha-beta unsaturated aldehydes;

c) Less than 40 wt.-% of components having a ClogP from 2.5 to 3.0 and less than 5 wt.-% of components having a ClogP of less than 2.5;

d) Less than 30 wt.-% of components having a molar volume of from 150 $cm^3$/mol to 170 $cm^3$/mol and less than 5 wt.-% of components having a molar volume of less than 150 $cm^3$/mol.

e) 50 wt.-% or less of components having Hansen solubility parameters $\delta D$, $\delta P$, and $\delta H$ fulfilling the conditions given by Equation 8:

$$\sqrt{4(\delta D - 19.077)^2 + (\delta P - 10.76)^2 + (\delta H - 4.81)^2} > 10$$

(Equation 8).

Alpha-beta unsaturated aldehydes that are particularly undesired in the second part of oil phase include (E)-dodec-2-enal (Aldehyde Mandarine); (Z)-2-benzylideneheptanal (Amyl Cinnamic Aldehyde); (2E)-3-phenylprop-2-enal (Cinnamic Aldehyde); (E)-3,7-dimethylocta-2,6-dienal (Citral); (E)-dodec-2-enal (Dodecenal); (E)-hex-2-enal (Hexenal-2-Trans); (E)-2-benzylideneoctanal (Hexyl Cinnamic Aldehyde); non-2-enal (Iris Aldehyde); methyl 2-aminobenzoate (Methyl Anthranilate); (Z)-2-methyl-3-phenylacrylaldehyde (Methyl Cinnamic Aldehyde); (E)-5-methyl-2-phenylhex-2-enal and (Z)-5-methyl-2-phenylhex-2-enal (Methyl Phenyl Hexenal); (2E,6Z)-nona-2,6-dienal (Nonadienal); 2,6,6-trimethylcyclohexa-1,3-dienecarbaldehyde (e.g. Safranal); (E)-tridec-2-enal (Tridecenal-2-Trans); and mixtures thereof, Schiff bases that are susceptible to release alpha-beta unsaturated aldehydes, such as (E)-methyl 2-(((4-(4-hydroxy-4-methylpentyl)cyclohex-3-en-1-yl)methylene)amino)benzoate (Lyranthion); methyl 2-((E)-((E)-2-benzylideneheptylidene)-amino)benzoate (Seringone) and (E)-methyl 2-((3-(4-(tert-butyl)phenyl)-2-methylprop-1-en-1-yl)amino)benzoate (Verdantion) are also undesired in the second oil phase.

In particular embodiments of the present invention, the second part of the oil phase comprises at least one fragrance ingredient. Encapsulated perfumes according to the present invention preferably comprise fragrance ingredients selected from the group consisting of (E)-2-methoxy-4-(prop-1-en-1-yl)phenyl acetate (Acetyl Isoeugenol); (2-(1-ethoxyethoxy)ethyl)benzene (Acetal E); (2-(1-propoxyethoxy)ethyl)benzene (Acetal R); octyl acetate (Acetate C 8 Octylic); (Z)-4,11,11-trimethyl-8-methylenebicyclo[7.2.0]undec-4-ene (Acetyl Caryophyllene); 2,6,10-trimethylundec-9-enal (Adoxal); N-ethyl-N-(m-tolyl)propionamide (Agarbois); 2-(tert-butyl)cyclohexyl acetate (Agrumex); decan-1-ol (Alcohol C 10 Decylic); dodecan-1-ol (Alcohol C 12 Lauric); tridecan-1-ol (Alcohol C 13 Oxo); decanal (Aldehyde C 10 Decylic); 2-methyldecanal (Aldehyde C 11 MOA); undec-10-enal (Aldehyde C 11 Undecylenic); undecanal (Aldehyde C 11); dodecanal (Aldehyde C 12 Lauric); 2-methylundecanal (Aldehyde C 12 MNA); (E)-undec-9-enal (Aldehyde Iso C 11); (E)-dodec-2-enal (Aldehyde Mandarine 10%/Tec); 2-methyl-4-(5,6,6-trimethylbicyclo[2.2.1]hept-2-yl-cyclohexanone (Aldrone); 2,6-dimethylheptan-4-yl acetate (Alicate); prop-2-enyl 2-(3-methylbutoxy)acetate (Allyl Amyl Glycolate); prop-2-enyl 3-cyclohexylpropanoate (Allyl Cyclohexyl Propionate); prop-2-enyl heptanoate (Allyl Oenanthate); 1-((2-(tert-butyl)cyclohexyl)oxy)butan-2-ol (Amber Core); 3,8,8,11a-tetramethyldodecahydro-1H-3,5a-epoxynaphtho[2,1-c]oxepine (Amberketal); (Z)-oxacycloheptadec-10-en-2-one (Ambrettolide); 2,5,5-trimethyl-1,2,3,4,4a,5,6,7-octahydronaphthalen-2-ol (Ambrinol); (4aR,5R,7aS,9R)-Octahydro-2,2,5,8,8,9a-hexamethyl-4H-4a,9-methanoazuleno[5,6-d]-1,3-dioxole (Ambrocenide Crystals); (3aR,5aS,9aS,9bR)-3a,6,6,9a-tetramethyl-2,4,5,5a,7,8,9,9b-octahydro-1H-benzo[e][1]benzofuran (Ambrofix); (3aR,5aS,9aS,9bR)-3a,6,6,9a-tetramethyl-2,4,5,5a,7,8,9,9b-octahydro-1H-benzo[e][1]benzofuran (Ambroxan); (Z)-2-benzylideneheptanal (Amyl Cinnamic Aldehyde); pentyl 2-phenylacetate (Amyl Phenyl Acetate); pentyl 2-hydroxybenzoate (Amyl Salicylate); phenylethyl 2-methylbutanoate (Anatolyl); (2-(isopentyloxy)ethyl)benzene (Anther); 1-(3,3-dimethylcyclohexyl)ethyl formate (Aphermate); (E)-methyl 2-((7-hydroxy-3,7-dimethyloctylidene)amino)benzoate (Aurantiol Pure); 3,5-diethyl-2,5-dimethylcyclohex-2-enone (Azarbre); 7-isopentyl-2H-benzo[b][1,4]dioxepin-3(4H)-one (Azurone); (1R,2S,4R)-2'-isopropyl-1,7,7-trimethylspiro[bicyclo-[2.2.1]heptane-2,4'-[1,3]dioxane] (Belambre); benzyl benzoate (Benzyl Benzoate); benzyl butanoate (Benzyl Butyrate); benzyl 3-phenylprop-2-enoate (Benzyl Cinnamate); benzyl 2-methylpropanoate (Benzyl Isobutyrate); benzyl 3-methylbutanoate (Benzyl Isovalerate); benzyl 2-phenylacetate (Benzyl Phenyl Acetate); benzyl 2-hydroxybenzoate (Benzyl Salicylate); 2-methyl-6-methyleneoct-7-en-2-yl acetate (Bergamyl Acetate); (ethoxymethoxy)cyclododecane (Boisambrene Forte); (1S,2R,5R)-2-ethoxy-2,6,6-trimethyl-9-methylenebicyclo[3.3.1]nonane (Boisiris); (2S,4S)-1,7,7-trimethylbicyclo[2.2.1]heptan-2-yl acetate (Bornyl Acetate); 3-(4-(tert-butyl)phenyl)propanal (Bourgeonal); (1R,5S,E)-1,5-dimethylbicyclo-[3.2.1]octan-8-one oxime (Buccoxime); 1-butoxy-1-oxopropan-2-yl butanoate (Butyl Butyro Lactate); 4-(tert-butyl)cyclohexanol (Butyl Cyclohexanol Para); 4-(tert-butyl)cyclohexyl acetate (Butyl Cyclohexyl Acetate Para); 2-(2-methylpropyl)quinoline (Butyl Quinoline Secondary); 1,2,4-trimethoxy-5-propylbenzene (Calmode); (4Z)-4,11,11-trimethyl-8-methylenebicyclo(7.2.0)undec-4-ene (Caryophyllene); 1,1,2,3,3-pentamethyl-2,3,6,7-tetrahydro-1H-inden-4(5H)-one (Cashmeran); 5-tert-butyl-2-methyl-5-propyl-2H-furan (Cassyrane); ((1S,8aR)-1,4,4-trimethyl-2,3,3a,4,5,8-hexahydro-1H-5,8a-methanoazulen-6-yl)methanol (Cedrenol); (1S,6R,8aR)-1,4,4,6-tetramethyloctahydro-1H-5,8a-methanoazulen-6-ol (Cedrol); (4Z,8Z)-1,5,9-trimethyl-13-oxabicyclo[10.1.0]trideca-4,8-diene (Cedroxyde); (1S,6R,8aR)-1,4,4,6-tetramethyloctahydro-1H-5,8a-methanoazulen-6-yl acetate (Cedryl Acetate Crystals); (1R,6S,8aS)-6-methoxy-1,4,4,6-tetramethyloctahydro-1H-5,8a-methanoazulene (Cedryl Methyl Ether); methyl 2-(3-oxo-2-pentylcyclopentyl)acetate (Cepionate); 3a,6,6,9a-tetramethyl-2,4,5,5a,7,8,9,9b-octahydro-1H-benzo[e][1]benzofuran (Cetalox); 2-methyl-4-(2,6,6-trimethylcyclohex-2-en-1-yl)butanal (Cetonal); (E)-1-(2,6,6-trimethylcyclohex-2-en-1-yl)hepta-1,6-dien-3-one (Cetone V); 3-phenylprop-2-enyl 3-phenylprop-2-enoate (Cinnamyl Cinnamate); (E)-1,1-dimethoxy-3,7-dimethylocta-2,6-diene (Citral Dimethyl Acetal); 3,7-dimethyloct-6-enal (Citronellal Synthetic); 3,7-dimethyloct-6-en-1-ol (Citronellol); 3,7-dimethyloct-6-en-1-yl acetate (Citronellyl Acetate); 3,7-dimethyloct-6-en-1-yl ethyl oxalate (Citronellyl Ethoxalate); 3,7-dimethyloct-6-en-1-yl formate (Citronellyl Formate); 3,7-dimethyloct-6-en-1-yl 2-methylpropanoate (Citronellyl Isobutyrate); 3,7-dimethyloct-6-enenitrile (Citronellyl Nitrile); 2-((3,7-dimethyloct-6-en-1-yl)oxy)acetaldehyde (Citronellyl Oxyacetaldehyde); 3,7-dimethyloct-6-en-1-yl propanoate (Citronellyl Propionate); (Z)-cycloheptadec-9-enone (Civettone); 2,4,4,7-tetramethyloct-6-en-3-one (Claritone); dodecanenitrile (Clonal); 2-(tert-pentyl)cyclohexyl acetate (Coniferan); 4-cyclohexyl-2-methylbutan-2-ol (Coranol); 2-(2-mercaptopropan-2-yl)-5-methylcyclohexanone (Corps Cassis); (4S)-4,7,7-trimethyl-6-thiabicyclo[3.2.1]octane (Corps Pamplemousse); 2-(3-phenylpropyl)pyridine (Corps Racine Vs 10%/Tec); (Z)-3-methylcyclotetradec-5-enone (Cosmone); (4-methylphenyl) octanoate (Cresyl Caprylate Para); (4-methylphenyl) 2-methylpropanoate (Cresyl Isobutyrate Para); (4-methylphenyl) 2-phenylacetate (Cresyl Phenyl Acetate Para); ethyl 2,6,6-trimethylcyclohexa-1,3-diene-1-carboxylate (Cristalon); 2-(4-methylphenoxy)acetaldehyde (Curgix); 3-(4-isopropylphenyl)-2-methylpropanal (Cyclamen Aldehyde Extra); 8,8-dimethyl-1,2,3,4,5,6,7,8-octahydronaphthalene-2-carbaldehyde (Cyclemone A); allyl 2-(cyclohexyloxy)acetate (Cyclogalbanate); 2-cyclohexylethyl acetate (Cyclohexyl Ethyl Acetate); 3-(4-methylcyclohex-3-en-1-yl)butan-1-ol (Cyclomethylene Citronellol); 8,8-dimethyl-1,2,3,4,5,6,7,8-octahydronaphthalene-2-carbaldehyde (Cyclomyral); hexyl 2-methylbutanoate (Cydrane); methyl 1,4-dimethylcyclohexanecarboxylate (Cyprisate); (E)-1-(2,6,6-trimethylcyclohexa-1,3-dien-1-yl)but-2-en-1-one (Damascenone); (E)-1-(2,6,6-trimethylcyclohex-2-en-1-yl)but-2-en-1-one (Damascone Alpha); (E)-1-(2,6,6-trimethyl-1-cyclohexenyl)but-2-en-1-one (Damascone Beta); 1-(2,6,6-trimethyl-1-cyclohex-3-enyl)but-2-en-1-one (Damascone Delta); decanenitrile (Decanonitrile); 6-isopropyloctahydronaphthalen-2(1H)-one (Decatone); (Z)-dec-4-enal (Decen-1-Al, Cis-4-); (E)-dec-2-enal (Decenal-2-Trans); (E)-dec-4-enal (Decenal-4-Trans); 9-decenal (Decenal-9); (oxybis(methylene))dibenzene (Dibenzyl Ether); 2-(sec-butyl)-1-vinylcyclohexyl acetate (Dihydro Ambrate); (Z)-3,7,11-trimethyldodeca-6,10-dienal (Dihydro Farnesal); 4-(2,6,6-trimethylcyclohex-1-en-1-yl)butan-2-one (Dihydro Ionone Beta); 3-methyl-2-pentylcyclopent-2-enone (Dihydro Jasmone); 3,7-dimethyloct-6-en-3-ol (Dihydro Linalool); 2,6-dimethyloct-7-en-2-ol (Dihydro Myrcenol); 2,6-dimethyloct-7-en-2-yl acetate (Dihydro Myrcenyl Acetate); 2-(4-methylcyclohexyl)propan-2-ol (Dihydro Terpineol); 2-methyl-1-phenylpropan-2-yl acetate (Dimethyl Benzyl Carbinyl Acetate); 2-methyl-1-phenylpropan-2-yl butanoate (Dimethyl Benzyl Carbinyl Butyrate); 4,7-dimethyloct-6-en-3-one (Dimethyl Octenone); 2,6-dimethyloct-7-en-2-ol (Dimyrcetol); 2-(2-(3,3,5-trimethylcyclohexyl)acetyl)cyclopentanone (Dione); 6-heptyltetrahydro-2H-pyran-2-one (Dodecalactone Delta); 5-octyloxolan-2-one (Dodecalactone Gamma); (E)-dodec-2-enal (Dodecenal 10%/Tec); (E)-4-((3aS,7aS)-hexahydro-1H-4,7-methanoinden-5(6H)-ylidene)butanal (Dupical); (E)-3-methyl-5-(2,2,3-trimethylcyclopent-3-en-1-yl)pent-4-en-2-ol (Ebanol); 3-(1-ethoxyethoxy)-3,7-dimethylocta-1,6-diene (Elintaal); 3-(1-ethoxyethoxy)-3,7-dimethylocta-1,6-diene (Elintaal Forte); (2E,4Z)-ethyl deca-2,4-dienoate (Ethyl Decadienoate); (E)-3,7-dimethylnona-1,6-dien-3-ol (Ethyl Linalool); (Z)-3,7-dimethylnona-1,6-dien-3-yl acetate (Ethyl Linalyl Acetate); ethyl octanoate (Ethyl Octanoate); ethyl nonanoate (Ethyl Pelargonate); ethyl 2,6,6-trimethylcyclohexa-1,3-diene-1-carboxylate (Ethyl Safranate); 1,4-dioxacycloheptadecane-5,17-dione (Ethylene Brassylate); methyl 2,4-dihydroxy-3,6-dimethylbenzoate (Evernyl); (3E,6E)-3,7,11-trimethyldodeca-1,3,6,10-tetraene (Farnesene); (2E,6Z)-3,7,11-trimethyldodeca-2,6,10-trien-1-ol (Farnesol Synthetic); (2S)-1,3,3-trimethylbicyclo[2.2.1]heptan-2-yl acetate (Fenchyl Acetate); 3a,6,6,9a-tetramethyldodecahydronaphtho[2,1-b]furan (Fixambrene); 1-(3,5,5,6,8,8-hexamethyl-5,6,7,8-tetrahydronaphthalen-2-yl)ethanone (Fixolide); 3-(4-ethylphenyl)-2,2-dimethylpropanenitrile (Fleuranil); 3-(4-ethylphenyl)-2,2-dimethylpropanal (Floralozone); 2,6-dimethyloct-7-en-2-ol (Floralym); 2-(tert-butyl)cyclohexyl ethyl carbonate (Floramat); 3-(3-isopropylphenyl)butanal (Florhydral); (E)-undec-9-enenitrile (Floridile); (3aR,6S,7aS)-3a,4,5,6,7,7a-hexahydro-1H-4,7-methanoinden-6-yl propanoate (Florocyclene); 2,4,6-trimethyl-4-phenyl-1,3-dioxane (Floropal); (Z)-1-(cyclooct-3-en-1-yl)propan-1-ol (Florymoss); 4,4,8,8-tetramethyloctahydro-4a,7-methanonaphtho[1,8a-b]oxirene (Folenox); 3-methyldodecanenitrile (Frescile); (3aS,4S,7R,7aS)-ethyl octahydro-1H-4,7-methanoindene-3a-carboxylate (Fruitate); 2-methyldecanenitrile (Frutonile); 4,6,6,7,8,8-hexamethyl-1,3,4,6,7,8-hexahydrocyclopenta[g]isochromene (Galaxolide); (3aR,6S,7aS)-3a,4,5,6,7,7a-hexahydro-1H-4,7-methanoinden-6-yl 2-methyl propanoate (Gardocyclene); 1-(1,2,8,8-tetramethyl-1,2,3,4,5,6,7,8-octahydronaphthalen-2-yl)ethanone (Georgywood); (E)-3,7-dimethylocta-2,6-dien-1-ol (Geraniol 980); (E)-3,7-dimethylocta-2,6-dien-1-yl acetate (Geranyl Acetate); (E)-6,10-dimethylundeca-5,9-dien-2-one (Geranyl Acetone); (E)-3,7-dimethylocta-2,6-dien-1-yl formate (Geranyl Formate); ethyl 2-ethyl-6,6-dimethylcyclohex-2-enecarboxylate (Givescone); 2-(8-isopropyl-6-methylbicyclo[2.2.2]oct-5-en-2-yl)-1,3-dioxolane (Glycolierral); 3a-ethyl-6,6,9a-trimethyldodecahydronaphtho[1,2-c]furan (Grisalva); 2-(3,8-dimethyl-1,2,3,4,5,6,7,8-octahydroazulen-5-yl)propan-2-yl acetate (Guaiyl Acetate); 2-butyl-4,6-dimethyl-3,6-dihydro-2H-pyran (Gyrane); (E)-oxacyclohexadec-12-en-2-one (Habanolide); methyl 3-oxo-2-pentylcyclopentaneacetate (Hedione); [2-[1-(3,3-dimethylcyclohexyl)ethoxy]-2-methylpropyl]propanoate (Helvetolide); heptan-2-one (Heptone); (2S)-ethyl 3-isopropylbicyclo[2.2.1]hept-5-ene-2-carboxylate (Herbanate); (3R,5R)-3-ethoxy-1,1,5-trimethylcyclohexane (Herbavert); 2-butyl-4,4,6-trimethyl-1,3-dioxane (Herboxane); (Z)-hex-3-en-1-yl benzoate (Hexenyl-3-Cis Benzoate); (Z)-hex-3-en-1-yl butanoate (Hexenyl-3-Cis Butyrate); (Z)-hex-3-en-1-yl (Z)-hex-3-enoate (Hexenyl-3-Cis Hexenoate); (Z)-hex-3-en-1-yl 2-methylpropanoate (Hexenyl-3-Cis Isobutyrate); (Z)-hex-3-en-1-yl 2-methyl butanoate (Hexenyl-3-Cis Methyl-2-Butyrate); (z)-hex-3-en-1-yl 2-hydroxybenzoate (Hexenyl-3-Cis Salicylate); (Z)-hex-3-enyl] (E)-2-methylbut-2-enoate (Hexenyl-3-Cis Tiglate); hexyl butanoate (Hexyl Butyrate); (E)-2-benzylideneoctanal (Hexyl Cinnamic Aldehyde); hexyl 2-methylpropanoate (Hexyl Isobutyrate); hexyl 2-hydroxybenzoate (Hexyl Salicylate); 8,8-di(1H-indol-3-yl)-2,6-dimethyloctan-2-ol (Indolene); (E)-4-(2,6,6-trimethylcyclohex-1-en-1-yl)but-3-en-2-one (Ionone Beta); (E)-3-methyl-4-(2,6,6-trimethylcyclohex-2-en-1-yl)but-3-en-2-one (Irisantheme); (E)-4-(2,6,6-trimethylcyclohex-2-en-1-yl)but-3-en-2-one (Irisone Alpha); (E)-4-(2,5,6,6-tetramethylcyclohex-2-en-1-yl)but-3-en-2-one (Irone Alpha); 1-(2,3,8,8-tetramethyl-1,2,3,4,5,6,7,8-octahydronaphthalen-2-yl)ethanone (Iso E Super); 2-methylpropyl benzoate (Isobutyl Benzoate); 2-methylpropyl 2-phenylacetate (Isobutyl Phenyl Acetate); 6-butan-2-yl-quinoline (Isobutyl Quinoline-2); 2-methylpropyl 2-hydroxybenzoate (Isobutyl Salicylate); 2-methoxy-3-(4-methylpentyl)pyrazine (Isohexylmethoxy Pyrazine); 2-hexylcyclopent-2-en-1-one (Isojasmone B 11); 2-hexylcyclopent-2-enone (Isojasmone T); 2,2,7,7-tetramethyltricyclo[6.2.1.01,6]undecan-5-one (Isolongifolanone); 3,5,5-trimethylhexyl acetate (Isononanyl Acetate Pure); 3-methylbutyl 3-methylbutanoate (Isopentyl Isovalerate); 4-methylpent-4-en-2-yl 2-methylpropanoate (Isopentyrate); (E)-3-methyl-4-(2,6,6-trimethylcyclohex-2-en-1-yl)but-3-en-2-one (Isoraldeine 70); (E)-3-methyl-4-(2,6,6-trimethylcyclohex-2-en-1-yl)but-3-en-2-one (Isoraldeine Cetone Alpha); (3aR,6S,7aS)-3a,4,5,6,7,7a-hexahydro-1H-4,7-methanoinden-6-yl acetate (Jasmacyclene); 2-hexylcyclopentanone (Jasmatone); (Z)-3-methyl-2-(pent-2-en-1-yl)cyclopent-2-enone (Jasmone Cis); (1-methyl-2-((1,2,2-trimethylbicyclo[3.1.0]hexan-3-yl)methyl)cyclopropyl)methanol (Javanol); 1-(4-methoxy-2,2,6,6-tetramethylcyclohex-3-en-1-yl)ethanone (Kefarene); 4-(1-ethoxyethenyl)-3,3,5,5-tetramethylcyclohexan-1-one (Kephalis); (Z)-3,4,5,6,6-pentamethylhept-3-en-2-one (Koavone); 3,4,5,6,6-pentamethylheptan-2-ol (Kohinool); (3E,6E)-2,4,4,7-tetramethylnona-6,8-dien-3-one oxime (Labienoxime); 2,8,8-trimethyloctahydro-1H-4a,2-(epoxymethano)naphthalen-10-one (Lactoscatone); 8-isopropyl-1-oxaspiro[4.5]decan-2-one (Laitone); (Z)-1-(1-ethoxyethoxy)hex-3-ene (Leaf Acetal); (2E,6Z)-3,7-dimethylnona-2,6-dienenitrile (Lemonile); 3-(4-(tert-butyl)phenyl)-2-methylpropanal (Lilial); 3,7-dimethylocta-1,6-dien-3-ol (Linalool Synthetic); 3,7-dimethylocta-1,6-dien-3-yl acetate (Linalyl Acetate Synthetic); 3,7-dimethylocta-1,6-dien-3-yl 3-phenylprop-2-enoate (Linalyl Cinnamate); 3,7-dimethylocta-1,6-dien-3-yl formate (Linalyl Formate); 3,7-dimethylocta-1,6-dien-3-yl 2-methylpropanoate (Linalyl Isobutyrate); 3,7-dimethylocta-1,6-dien-3-yl propanoate (Linalyl Propionate); (3R,3aR,8R,8aS)-4,4,8-trimethyl-9-methylenedecahydro-3,8-methanoazulene (Longifolene Std); bicyclo[2.2.2]oct-5-ene-2-carboxaldehyde (Maceal); 2,2-dimethyl-3-(m-tolyl)propan-1-ol (Majantol); 2-(4-(tert-butyl)phenyl)acetonitrile (Marenil); 3-methyl-5-phenylpentanal (Mefranal); 3-methyl-5-phenylpentan-1-ol (Mefrosol); 2-(4-methylcyclohexyl)propan-2-yl acetate (Menthanyl Acetate); 2-isopropyl-5-methylcyclohexanol (Menthol Dl); 2-(4-methylcyclohex-3-en-1-yl)propane-2-thiol (Mercapto-8 Menthene-1 Para); (2-butan-2-yl-1-methylcyclohexyl) acetate (Metambrate); butyl 2-methylpentanoate (Methyl Camomille);

1-((1S,8aS)-1,4,4,6-tetramethyl-2,3,3a,4,5,8-hexahydro-1H-5,8a-methanoazulen-7-yl)ethanone (Methyl Cedryl Ketone); 5-hexyl-5-methyloxolan-2-one (Methyl Decalactone Gamma); methyl 2-hexyl-3-oxocyclopentane-1-carboxylate (Methyl Dihydro Isojasmonate); (E)-1,2-dimethoxy-4-(prop-1-en-1-yl)benzene (Methyl Isoeugenol); methyl non-2-ynoate (Methyl Octyne Carbonate); 6,6-dimethoxy-2,5,5-trimethylhex-2-ene (Methyl Pamplemousse); (E)-3-methyl-4-(2,6,6-trimethylcyclohex-1-en-1-yl)but-3-en-2-one (Methylionantheme); 1a,3,3,4,6,6-hexamethyl-1a,2,3,4,5,6,7,7a-octahydronaphtho[2,3-b]oxirene (Moxalone); (Z)-3-methylcyclopentadec-5-enone (Muscenone); 3-methylcyclopentadecanone (Muscone); 1,7-dioxacycloheptadecan-8-one (Musk R1); 4-(4-methylpent-3-en-1-yl)cyclohex-3-enecarbaldehyde (Myraldene); (4-(4-methylpent-3-en-1-yl)cyclohex-3-en-1-yl)methyl acetate (Myraldyl Acetate); 7-methyl-3-methyleneocta-1,6-diene (Myrcene 90); (2-methyl-6-methylideneoct-7-en-2-yl) acetate (Myrcenyl Acetate); 2-methylundecanoic acid (Mystikal); 2-methylundecanoic acid (Mystikal); 2-(2-(4-methylcyclohex-3-en-1-yl)propyl)cyclopentan-1-one (Nectaryl); 2-methyl-6-methyleneoct-7-en-2-yl acetate (Neobergamate Forte); 10-isopropyl-2,7-dimethyl-1-oxaspiro[4.5]deca-3,6-diene (Neocaspirene); (E)-methyl non-2-enoate (Neofolione); (2Z)-3,7-dimethylocta-2,6-dien-1-ol (Nerolex); (E)-3,7,11-trimethyldodeca-1,6,10-trien-3-ol (Nerolidol Synthetic); (Z)-3,7-dimethylocta-2,6-dien-1-yl acetate (Neryl Acetate Hc); 1-(2,2,6-trimethylcyclohexyl)hexan-3-ol (Nimberol); (E)-13-methyloxacyclopentadec-10-en-2-one (Nirvanolide); (2Z,6E)-2,6-nonadien-1-yl acetate (Nonadienyl Acetate); 6,8-dimethylnonan-2-ol (Nonadyl); 4,4a-dimethyl-6-(prop-1-en-2-yl)-4,4a,5,6,7,8-hexahydronaphthalen-2(3H)-one (Nootkatone Crystals); 2-(6,6-dimethylbicyclo[3.1.1]hept-2-en-2-yl)ethyl acetate (Nopyl Acetate); 2,4-dimethyl-2-(5,5,8,8-tetramethyl-5,6,7,8-tetrahydronaphthalen-2-yl)-1,3-dioxolane (Okoumal); 7-isopropyl-8,8-dimethyl-6,10-dioxaspiro[4.5]decane (Opalal); 4-(2-methoxypropan-2-yl)-1-methylcyclohexene (Orange Flower Ether); 4-(tert-pentyl)cyclohexanone (Orivone); 2,4a,5,8a-tetramethyl-1,2,3,4,4a,7,8,8a-octahydronaphthalen-1-yl formate (Oxyoctaline Formate); 2-ethyl-N-methyl-N-(m-tolyl)butanamide (Paradisamide); 1,1-dimethoxynon-2-yne (Parmavert); 5-heptyldihydrofuran-2(3H)-one (Peach Pure); 2-methyl-4-methylene-6-phenyltetrahydro-2H-pyran (Pelargene); 3,7-dimethyloctan-1-ol (Pelargol); 2-cyclohexylidene-2-phenylacetonitrile (Peonile); 3,7-dimethylocta-1,6-dien-3-yl dimethylcarbamate (Pepperwood); 2-methylpentyl 2-methylpentanoate (Peranat); 2-cyclohexylhepta-1,6-dien-3-one (Pharaone); 2-(phenoxy)ethyl 2-methylpropanoate (Phenoxy Ethyl Isobutyrate); 2-phenylethyl 3-phenylprop-2-enoate (Phenyl Ethyl Cinnamate); 2-phenylethyl 2-methylpropanoate (Phenyl Ethyl Isobutyrate); 2-phenylethyl 2-phenylacetate (Phenyl Ethyl Phenyl Acetate); 2-phenylethyl 2-hydroxybenzoate (Phenyl Ethyl Salicylate Crystals); 3-phenylpropyl acetate (Phenyl Propyl Acetate); 3-(6,6-dimethylbicyclo[3.1.1]hept-2-en-2-yl)propanal (Pinoacetaldehyde); (3aR,6S,7aS)-3a,4,5,6,7,7a-hexahydro-1H-4,7-methanoinden-6-yl 2,2-dimethylpropanoate (Pivacyclene); 2,2-dimethyl-2-pheylethyl propanoate (Pivarose); (4aS,8aR)-7-methyloctahydro-1,4-methanonaphthalen-6(2H)-one (Plicatone); ethyl 2-cyclohexylpropanoate (Poirenate); (2E,5E)-5,6,7-trimethylocta-2,5-dien-4-one (Pomarose); 1-methyl-4-(4-methylpent-3-en-1-yl)cyclohex-3-enecarbaldehyde (Precyclemone B); (E)-2-ethoxy-5-(prop-1-en-1-yl)phenol (Propenyl Guaethol (Vanitrope)); 6-(sec-butyl)quinoline (Pyralone); (E)-2-ethyl-4-(2,2,3-trimethylcyclopent-3-en-1-yl)but-2-en-1-ol (Radjanol); 2-(cyclohexylmethyl)-4,4,6-trimethyl-1,3-dioxane (Resedal); mixture of 3,7-dimethyloct-6-en-1-ol and 3,7-dimethyloct-7-en-1-ol (Rhodinol); 2,4-dimethyl-4-phenyltetrahydrofuran (Rhubafuran); (2R,8aS)-3',6-dimethyl-3,4,4a,5,8,8a-hexahydro-1H-spiro[1,4-methanonaphthalene-2,2'-oxirane] (Rhubofix); (4aR,8aS,E)-6-ethylideneoctahydro-2H-5,8-methanochromene (Rhuboflor); 2,2,2-trichloro-1-phenylethyl acetate (Rosacetol); dec-9-en-1-ol (Rosalva); 3-(2-methylpropyl)-1-methylcyclohexanol (Rossitol); 4-methyl-2-phenyl-3,6-dihydro-2H-pyran (Rosyrane Super); 1,1-diethoxycyclohexane (Rum Acetal); 2,3,3-trimethyl-1-indanone (Safraleine); 3-((1R,2S,4R,6R)-5,5,6-trimethylbicyclo[2.2.1]heptan-2-yl)cyclohexanol (Sandela Concentrated); 4,5,6,7,8,9,10,11,12,13-decahydrocyclododeca[d]oxazole (Sclarene); 2-(1-(3,3-dimethylcyclohexyl)ethoxy)-2-methylpropyl cyclopropanecarboxylate (Serenolide); cyclopentadecanone, hexadecanolide (Silvanone Supra); 2-methyl-3-[4-(2-methylpropyl)phenyl]propanal (Silvial); 2',2',3,7,7-pentamethylspiro[bicyclo[4.1.0]heptane-2,5'-[1,3]dioxane] (Spirambrene); 1-(spiro[4.5]dec-6-en-7-yl)pent-4-en-1-one (Spirogalbanone); ethyl methyl phenyl glycidate (Strawberry Pure); 1-phenylethyl propanoate (Styrallyl Propionate); (E)-6-ethyl-3-methyloct-6-en-1-ol (Super Muguet); 2-(heptan-3-yl)-1,3-dioxolane (Syvertal); (E)-6,10-dimethylundeca-5,9-dien-2-yl acetate (Tangerinol); 2-(4-methyl-1-cyclohex-3-enyl)propan-2-yl acetate (Terpinyl Acetate); 3,7-dimethyloctanal (Tetrahydro Citral); 3,7-dimethyloctan-3-ol (Tetrahydro Linalool); 3,7-dimethyloctan-3-yl acetate (Tetrahydro Linalyl Acetate); 2,6-dimethyloctan-2-ol (Tetrahydro Myrcenol); oxacyclohexadecan-2-one (Thibetolide); (E)-3,7-dimethylocta-2,6-diene-1-thiol (Thiogeraniol); 2,2,6-trimethylcyclohexyl-3-hexanol (Timberol); (E)-tridec-2-enenitrile (Tridecene-2-Nitrile); 1-((2E,5Z,9Z)-2,7,8-trimethylcyclododeca-2,5,9-trien-1-yl)ethanone (Trimofix O); 6-hexyltetrahydro-2H-pyran-2-one (Undecalactone Delta); undecan-2-one (Methyl Nonyl Ketone); (3E,5Z)-undeca-1,3,5-triene (Undecatriene); (E)-4-methyldec-3-en-5-ol (Undecavertol); (E)-undec-2-enenitrile (Undecene 2 Nitrile); 2,2,5-trimethyl-5-pentylcyclopentanone (Veloutone); (Z)-cyclohexadec-5-enone (Velvione); (E)-methyl 2-((3-(4-(tert-butyl)phenyl)-2-methylprop-1-en-1-yl)amino)benzoate (Verdantiol); (2-(1-ethoxyethoxy)ethyl)benzene (Verdilyn); 2-(tert-butyl)cyclohexanol (Verdol); 1-methyl-4-(4-methylpentyl)cyclohex-3-enecarbaldehyde (Vernaldehyde); 1-((1S,8aS)-1,4,4,6-tetramethyl-2,3,3a,4,5,8-hexahydro-1H-5,8a-methanoazulen-7-yl)ethanone (Vertofix Coeur); 2,4-diethoxy-5-methylpyrimidine (Vethymine); 4-methyl-4-phenylpentan-2-yl acetate (Vetikol Acetate/Corps Rhubarb); (2R,5R,8S)-4,4,8-trimethyltricyclo[6.3.1.02,5]dodecan-1-yl acetate (Vetynal); [(3Z)-4,11,11-trimethyl-8-methylidene-5-bicyclo[7.2.0]undec-3-enyl] acetate (Vetyvenal); undec-10-enenitrile (Violiff); 2-(2,4-dimethylcyclohexyl)pyridine (Zinarine); cedar wood oileucalyptus oil; galbanum oil; clove oillavandin and lavender oil mandarin oilorange terpenespatchouli oilylang oil; and mixtures thereof. These fragrance ingredients are particularly suitable for obtaining stable and performing microcapsules, owing to their favorable lipophilicity and olfactive performance.

Ingredients that fulfill the requirements defined hereinabove for the first part of the oil phase (GROUP 1 or GROUP 2) may be used in both oil phases.

The second part of the oil phase may also comprise one or more precursors, meaning a material that is capable of releasing a fragrance ingredient by the means of a stimulus, such as a change of temperature, the presence of oxidants, the action of enzymes or the action of light, provided this precursor is not or does not release a alpha-beta unsaturated aldehyde. Such pro-fragrances are well-known to the art.

The oil cores may also comprise at least one functional cosmetic ingredient. The functional cosmetic ingredients for use in the encapsulated composition are preferably hydrophobic

Particularly useful functional cosmetic ingredients may be selected from the group consisting of emollients, smoothening ingredients, hydrating ingredients, soothing and relaxing ingredients, decorative ingredients, deodorants, anti-aging ingredients, cell rejuvenating ingredients, draining ingredients, remodeling ingredients, skin levelling ingredients, preservatives, anti-oxidants, antibacterial or bacteriostatic ingredients, cleansing ingredients, lubricating ingredients, structuring ingredients, hair conditioning ingredients, whitening ingredients, texturing ingredients, softening ingredients, anti-dandruff ingredients, and exfoliating ingredients.

Particularly useful functional cosmetic ingredients include, but are not limited to hydrophobic polymers, such as alkyldimethylsiloxanes, polymethylsil-sesquioxanes, polyethylene, polyisobutylene, styrene-ethylene-styrene and styrene-butylene-styrene block copolymers, and the like; mineral oils, such as hydrogenated isoparaffins, silicone oils and the like; vegetable oils, such as argan oil, jojoba oil, aloe vera oil, and the like; fatty acids and fatty alcohols and their esters; glycolipides; phospholipides; sphingolipides, such as ceramides; sterols and steroids; terpenes, sesquiterpenes, triterpenes and their derivatives; essential oils, such as Arnica oil, Artemisia oil, Bark tree oil, Birch leaf oil, Calendula oil, Cinnamon oil, Echinacea oil, Eucalyptus oil, Ginseng oil, Jujube oil, Helianthus oil, Jasmine oil, Lavender oil, Lotus seed oil, Perilla oil, Rosmary oil, Sandal wood oil, Tea tree oil, Thyme oil, Valerian oil, Wormwood oil, Ylang Ylang oil, and Yucca oil.

In particular, the at least one functional cosmetic ingredient may be selected from the group consisting of Sandal wood oil, such as Fusanus Spicatus kernel oil; Panthenyl triacetate; Tocopheryl acetate; Tocopherol; Naringinin; Ethyl linoleate; Farnesyl acetate; Farnesol; Citronellyl methyl crotonate; and Ceramide-2 (1-Stearoiyl-C18-Sphingosine, CAS-No: 100403-19-8).

In respect to step b), the second part of the oil phase is preferably added after the formation of the macromer has been completed.

In particular embodiments, the complex coacervate comprised in the composition according to present invention, comprises a cationic macromolecule selected from gelatin, chitosan and permanently charged cationic polysaccharide, such as cationic hydroxypropyltrimonium starch or hydroxypropyltrimonium guar gum, and mixtures thereof, and an anionic polysaccharide selected from the group consisting of pectin, gum Arabic and alginate, and their sodium, potassium, magnesium or calcium carboxylate salts, and mixtures thereof. Preferably, the anionic polysaccharide is pectin.

In preferred embodiments of the present invention, the negatively charged polyelectrolyte comprised in the coacervate is the polymeric surfactant comprised in the aqueous phase of the composition.

In preferred embodiments, the coacervate to polymeric stabilizer weight ratio in a composition according to the invention is from 1 to 3, more particularly from 1.5 to 2.0. Such a ratio provides a desirable balance between the stability of the microcapsules with respect to retention of the benefit agent in the microcapsule over time and shell biodegradability.

In particular embodiments of the present invention, the complex coacervate and the polymeric stabilizer are cross-linked with a polyfunctional aldehyde, a polyfunctional gylcidyl compound, a polyfunctional succinimidyl compound or an enzyme; and with unreacted isocyanate groups still present on the polymeric stabilizer.

The composition according to the invention may be rendered in various physical forms, for example in the form of a slurry, a powder, a granulates, in the form of flakes or an extrudate.

Compositions according to the invention in the form of liquid slurries may comprise from 10 to 50 wt.-%, more particularly from 15 to 25 wt.-%, of core-shell microcapsules.

Compositions according to the invention in solid form may comprise from 1 to 100 wt.-% of core-shell microcapsules. However, depending on the application or on the nature of the benefit agent, it may be preferable to limit or, in the contrary, to maximize the level of core-shell microcapsules in the solid form. For example, a limitation of the level of the core-shell microcapsules in the solid may be particularly desired if the encapsulated material is flammable, reactive, pungent or expensive.

Hence, the optimal level of encapsulated fragrance ingredients in a solid composition according to the invention may be less than 50 wt.-%, more particularly less than 35 wt.-% and still more particularly less than 20 wt.-%, or even less than 15 wt.-%, depending on the flammability of such fragrance ingredients and the associated explosion risks.

In yet another aspect, the present invention relates to a use of a composition according to the invention to

enhance the performance of a benefit agent, and particularly a fragrance composition, in a consumer product.

The present invention also relates to a consumer product comprising a composition according to the invention. The consumer product is preferably selected from the group consisting of fabric care detergents and conditioners, hair care conditioners, shampoos, heavy duty liquid detergents, hard surface cleaners, detergent powders, soaps, shower gels and skin care products.

Compositions according to the present invention are particularly useful when employed as perfume delivery vehicles in consumer goods that require, for delivering optimal perfumery benefits, that the microcapsules adhere well to a substrate on which they are applied. Such consumer goods include hair shampoos and conditioners, as well as textile-treatment products, such as laundry detergents and conditioners.

Particular features and further advantages of the present invention are described more fully in the following examples.

Example 1 - Fragrance compositions

A series of fragrances were prepared, the compositions of which are reported in Table 1. Table 1 shows the composition of the full fragrances and the way these fragrances were split in first and second parts of the oil phase.

Table 1 : Fragrance composition and fragrance split

| Fragrance 1.1 | | | | | | | | | |
|---|---|---|---|---|---|---|---|---|---|
| | dD | dP | dH | D | Molar Volume [cm3/mol] | ClogP | Total oil phase wt.-% | Oil phase 1st part wt.-% | Oil phase 2nd part wt.-% |
| ISO E SUPER | 16.90 | 3.30 | 2.40 | 8.99 | 246.3 | 4.9 | 20 | 15 | 5 |
| FLOROCYCLENE | 17.00 | 3.00 | 3.60 | 8.88 | 186.6 | 3.4 | 20 | 20 | |
| AGRUMEX | 15.90 | 1.90 | 2.90 | 11.07 | 213.1 | 4.1 | 10 | | 10 |
| ALDEHYDE C 12 MNA | 16.10 | 4.20 | 3.20 | 9.00 | 224.3 | 5.1 | 8 | | 8 |
| TETRAHYDRO LINALOOL | 15.90 | 2.60 | 6.00 | 10.41 | 191.5 | 3.5 | 12 | | 12 |
| EUCALYPTOL | 16.70 | 2.80 | 2.50 | 9.56 | 167.1 | 2.8 | 5 | | 5 |
| DAMASCONE DELTA | 16.90 | 3.50 | 2.60 | 8.75 | 216.0 | 3.6 | 5 | 5 | |
| ALPHA METHYL IONONE | 17.00 | 3.20 | 2.90 | 8.84 | 222.2 | 4.0 | 5 | 5 | |
| FLORALOZONE | 17.20 | 5.10 | 3.30 | 6.96 | 202.0 | 3.8 | 5 | | 5 |
| UNDECAVERTOL | 16.40 | 3.40 | 7.50 | 9.49 | 201.4 | 3.9 | 5 | | 5 |
| AMYL SALICYLATE | 17.50 | 6.60 | 7.00 | 5.66 | 191.7 | 4.5 | 5 | 5 | |
| TOTAL | | | | | | | 100 | 50 | 50 |

Fragrance 1.2

| | dD | dP | dH | D | Molar Volume [cm3/mol] | ClogP | Total oil phase wt.-% | Oil phase 1st part wt.-% | Oil phase 2nd part wt.-% |
|---|---|---|---|---|---|---|---|---|---|
| BENZYL SALICYLATE | 19.3 | 6.5 | 7.8 | 5.22 | 186.4 | 4.163 | 15 | 10 | 5 |
| TETRAHYDRO LINALOOL | 15.9 | 2.6 | 6 | 10.41 | 191.5 | 3.517 | 15 | | 15 |
| AGRUMEX | 15.9 | 1.9 | 2.9 | 11.07 | 213.1 | 4.059 | 15 | | 15 |
| ISO E SUPER | 16.9 | 3.3 | 2.4 | 8.97 | 246.3 | 4.847 | 10 | 10 | |
| HEDIONE | 17.1 | 5.5 | 4.6 | 6.58 | 229.8 | 2.912 | 6 | 6 | |
| CITRONELLOL EXTRA | 16.5 | 4 | 7.9 | 9.04 | 184.9 | 3.253 | 10 | | 10 |
| TRICYCLAL | 16.9 | 5 | 4.6 | 7.22 | 144.2 | 2.361 | 3 | | 3 |
| DIPENTENE | 16.7 | 1.9 | 3.2 | 10.18 | 163.2 | 4.352 | 7 | | 7 |
| DAMASCONE DELTA | 16.9 | 3.5 | 2.6 | 8.75 | 216.0 | 3.62 | 4 | 4 | |
| MANZANATE | 15.7 | 3.6 | 4.7 | 9.84 | 164.4 | 2.607 | 4 | | 4 |
| PEACH PURE | 16.4 | 9.2 | 4.2 | 5.61 | 179.9 | 2.89 | 2 | | 2 |
| DIMETHYL BENZYL CARBINYL ACETATE | 16.7 | 2.4 | 3.5 | 9.71 | 191.8 | 4.045 | 4 | | 4 |
| NECTARYL | 17.7 | 4.2 | 3.3 | 7.27 | 231.0 | 4.438 | 2 | | 2 |
| NYMPHEAL | 17.4 | 4.8 | 3.4 | 6.98 | 218.5 | 3.7 | 2 | 2 | |
| ROSYFOLIA | 16.7 | 3.5 | 6.8 | 8.90 | 201.7 | 5.5 | 1 | | 1 |
| | | | | | | | 100 | 32 | 68 |

Fragrance 1.3

| | dD | dP | dH | D | Molar Volume [cm3/mol] | ClogP | Total oil phase wt.-% | Oil phase 1st part wt.-% | Oil phase 2nd part wt.-% |
|---|---|---|---|---|---|---|---|---|---|
| ETHYLENE BRASSYLATE | 16.8 | 3.6 | 4.9 | 8.49 | 276.7 | 3.024 | 14 | 14 | |
| IONONE BETA | 17 | 3.5 | 3.2 | 8.52 | 203.6 | 3.77 | 10 | 10 | |
| CYCLAMEN ALDEHYDE | 17.4 | 4.9 | 3.3 | 6.92 | 203.0 | 3.829 | 2 | 2 | |
| PEONILE | 18.3 | 5.6 | 4.5 | 5.40 | 187.4 | 3.86 | 10 | 10 | |
| ETHYL METHYL-2-BUTYRATE | 15.6 | 3.8 | 4.9 | 9.84 | 147.9 | 2.078 | 2 | | 2 |
| ALPHA METHYL IONONE | 17 | 3.2 | 2.9 | 8.84 | 222.2 | 4.019 | 10 | 10 | |
| DECALACTONE GAMMA | 16.4 | 10 | 4.5 | 5.42 | 179.9 | 2.361 | 5 | 5 | |
| HEXYL ACETATE | 16 | 4.1 | 5.4 | 9.09 | 164.1 | 2.827 | 8 | | 8 |

(continued)

| | dD | dP | dH | D | Molar Volume [cm3/mol] | ClogP | Total oil phase wt.-% | Oil phase 1st part wt.-% | Oil phase 2nd part wt.-% |
|---|---|---|---|---|---|---|---|---|---|
| DAMASCONE ALPHA | 17 | 3.5 | 3.2 | 8.52 | 214.0 | 3.82 | 4 | 4 | |
| UNDECAVERTOL | 16.4 | 3.4 | 7.5 | 9.49 | 201.4 | 3.892 | 4 | | 4 |
| ALDEHYDE C 12 MNA | 16.1 | 4.2 | 3.2 | 9.00 | 224.3 | 5.066 | 2 | | 2 |
| PATCHOULI OIL DECOL INDONESIA ORPUR | | | | | | | 5 | | 5 |
| DIHYDRO MYRCENOL | 16 | 3.4 | 6.4 | 9.72 | 186.6 | 3.033 | 10 | | 10 |
| MAHONIAL | 16.6 | 6.2 | 6.5 | 6.94 | 218.5 | 1.8 | 2 | | 2 |
| ROSYFOLIA | 16.7 | 3.5 | 6.8 | 8.90 | 201.7 | 5.5 | 4 | | 4 |
| BELAMBRE | 16.8 | 2.8 | 1.5 | 9.75 | 251.4 | 4.426 | 8 | 5 | 3 |
| | | | | | | | 100 | 60 | 40 |

Example 2 - Formation of microcapsules comprising a hydrogel formed by the combination of pectin and gelatin (according to the present invention)

In Example 2.1, the microcapsules were obtained as follows:

a) A core composition was prepared by admixing 0.7 g of bipodal aminosilane (bis(3-triethoxysilylpropyl)amine), 0.48 g Takenate D-110N (ex Mitsui) and 19.25 g of the a first part of the first part of the oil phase of fragrance 1.1, corresponding to 50 wt.-% of the total fragrance composition;

b) The core composition was heated at 25 +/- 2 °C °C and maintained under stirring for 15 minutes;

c) After 15 minutes, 19.25 g of the second oil phase of fragrance 1 was added to the emulsion obtained in step c) while maintaining both temperature and stirring;

d) The core composition obtained in step b) was emulsified in a mixture of 1.0 g high methoxylated grade pectin (of type APA 104, ex Roeper) in 73.3 g of water by using a 300 ml reactor and a cross-beam stirrer with pitched beam operating at a stirring speed of 600 rpm at a temperature of 25 +/-2 °C for 10 min;

e) The temperature of the system was raised to 85 +/- 2 °C over 4 hours, 0.3 g of trimesic acid (1,3,5-benzenetricarboxylic acid) were added and the system was maintained at this temperature for 1.3 h while maintaining stirring as in step b);

f) The system was slowly cooled to 40 °C over a period of 2.25 hours, while maintaining stirring as in step b);

g) At a temperature of 40 +/- 2 °C, 10 g of a 10% gelatin solution in water was added, while maintaining stirring as in step b);

h) The system was slowly cooled to 10 °C over a period of 2.25 hours, while maintaining stirring as in step b);

i) Once the system had reached the temperature of 10 °C, 0.02 g of a 50 wt.-% glutaraldehyde solution in water was added and the system was maintained at this temperature for 1 hour, while maintaining stirring as in step b), in order to form a slurry of core-shell microcapsules;

j) The slurry of core-shell capsules obtained in step f) was finally let to stabilize at room temperature.

The solid content of the slurry obtained was 33.1 wt.-%, the volume median size (d50) of the capsules was 32 μm and the encapsulation efficiency of 99 %.

In Example 2.2, Fragrance 2 was used and the first to second part of the fragrance oil weight ratio was modified, i.e. 12.3 g of first part was used in step a) and 26.2 g of second part was used in step d). In Example 2.3, these amounts were 16.5 g and 22 g, respectively.

Example 3 - Formation of microcapsules comprising a hydrogel formed by the combination of pectin and gelatin (comparative example)

In Example 2.1, the microcapsules were obtained as follows:

a) A core composition was prepared by admixing 0.7 g of bipodal aminosilane (bis(3-triethoxysilylpropyl)amine), 0.48 g Takenate D-110N (ex Mitsui) and 38.5 g of fragrance composition 1, corresponding to 100 wt % of the fragrance 1 composition;

b) The core composition obtained in step a) was emulsified in a mixture of 1.0 g high methoxylated grade pectin (of type APA 104, ex Roeper) in 73.3 g of water by using a 300 ml reactor and a cross-beam stirrer with pitched beam operating at a stirring speed of 600 rpm at a temperature of 25 +/-2 °C for 10 min;

c) The temperature of the system was raised to 85 +/- 2 °C over a period of 4 hours, 0.3 g of trimesic acid (1,3,5-benzenetricarboxylic acid) were added and the system was maintained at this temperature for 1.3 h while maintaining stirring as in step b);

d) The system was slowly cooled to 40 °C over a period of 2.25 hours, while maintaining stirring as in step b);

e) At a temperature of 40 +/- 2 °C, 10 g of a 10% gelatin solution in water was added, while maintaining stirring as in step b);

f) The system was slowly cooled to 10 °C over a period of 2.25 hours, while maintaining stirring as in step b);

g) Once the system had reached the temperature of 10 °C, 0.02 g of a 50 wt.-% glutaraldehyde solution in water was added and the system was maintained at this temperature for 1 hour, while maintaining stirring as in step b), in order to form a slurry of core-shell microcapsules;

h) The slurry of core-shell capsules obtained in step f) was finally let to stabilize at room temperature.

The solid content of the slurry obtained was 33.1 wt.-%, the volume median size (d50) of the capsules was 32 μm and the encapsulation efficiency of 99 %.

In Example 3.2, the same procedure was applied, but with perfume 1.2 used in step a), whereas in Example 3.3, perfume 1.3 was used in step a)

Example 4 - Comparison of microcapsule performance

The performance of the capsules obtained in Example 1 and 2, was evaluated in terms of stability.

For stability assessment, the base was an unperfumed commercial proprietary laundry care conditioner base. For each assessment 1 wt.-% of slurry was dispersed in the base under stirring with a paddle mixer. The encapsulated core composition comprised additionally 0.02 wt.-% of Hostasol® Yellow 3G (Clariant) as fluorescent dye. The samples were then stored for 8 weeks at 37 °C. The leakage from the capsules was assessed visually by fluorescent light microscopy, operating at 488 nm excitation light wavelength and 515 nm emission light wavelength, according to the following scale:

- Poor stability: Collapsed microcapsules and fluorescent droplets are visible;
- Average stability: Partially collapsed microcapsules coexist with fluorescent droplets;
- Good stability: All capsules are still full of fluorescent core composition and no fluorescent droplets are visible.

| Example | Capsule stability In conditioner | Comments |
|---|---|---|
| 2.1 | Good | |
| 2.2 | Good | |
| 2.3 | Good | |
| 3.1 | Poor | The full fragrance contains<br>- 13 wt.-% aldehydes, i.e. significantly more than the max 5 wt.-% imposed by rule a) of claim 9;<br>- 22 wt.-% of ingredients having a D value larger than 10, i.e. significantly more than the max 1 wt,.% imposed by rule d) of claim 9. |
| 3.2 | Poor | The full fragrance contains<br>- 5 wt.-% aldehydes, i.e. at the limit of the range of max 5 wt.-% imposed by rule a) of claim 9;<br>- 37 wt.-% of ingredients having a D value larger than 10, i.e. significantly more than the max 1 wt,.% imposed by rule d) of claim 9. |
| 3.3 | Average | The content of aldehyde in the fragrance is slightly above the maximal limit according to rule a) of claim 9, but all other rules are fulfilled. |

**Claims**

**1.** A method of making a composition comprising a plurality of core-shell microcapsules, the method comprising the step of:

a) Providing an oil phase comprising a benefit agent to be encapsulated and a macromer formed by the reaction of one or more reactive building blocks;
b) providing an aqueous phase;
c) emulsifying the oil phase and the aqueous phase to form a dispersed phase consisting of a plurality of oil cores in a continuous aqueous phase;
d) polymerizing the macromer to form a polymeric stabilizer at the interface between the oil cores and the aqueous phase;
e) Causing a positively charged polyelectrolyte and a negatively charged polyelectrolyte, each independently added to the aqueous phase during step b), c) or d), to undergo a process of coacervation thereby to form a hydrated-polymer shell around the oil cores; and
f) Cross-linking the hydrated-polymer shell and the polymeric stabilizer in order to harden the shell around the oil cores, thereby to form a composition comprising a plurality of core-shell microcapsules.

**2.** The method according to claim 1, wherein the macromer is formed in-situ in the oil phase and the oil phase comprises, based on the total weight of the oil phase:

a) Less than 1 wt.-%, more particularly less than 0.1 wt.-%, and still more particularly 0.0 wt % of components susceptible of reacting with the reactive building blocks;
b) 40 wt.-% or more, more particularly 60 wt.-% or more, still more particularly 75 wt.-% or more still more particularly 90 wt.-% or more, still more particularly 95 wt.-% or more components having Hansen Solubility Parameters that fulfill Equation 1:

$$D = \sqrt{4(\delta D_A - \delta D_B)^2 + (\delta P_A - \delta P_B)^2 + (\delta H_A - \delta H_B)^2} < 9$$

(Equation 1)

c) Less than 60 wt.-%, more particularly less than 40 wt.-%, still more particularly less than 25 wt.-%, still more particularly less than 10 wt.-%, still more particularly less than 5 wt.-% of components having Hansen Solubility Parameters δD, δP, and δH fulfilling the conditions given by Equation 2, and referred to as GROUP 2 components:

$$9 < \sqrt{4(\delta D_A - \delta D_B)^2 + (\delta P_A - \delta P_B)^2 + (\delta H_A - \delta H_B)^2} \leq 10$$

(Equation 2);

and

d) less than 1 wt.-% of components, having Hansen Solubility Parameters δD, δP, and δH fulfilling the conditions given by Equation 3;

$$\sqrt{4(\delta D_A - \delta D_B)^2 + (\delta P_A - \delta P_B)^2 + (\delta H_A - \delta H_B)^2} > 10$$

(Equation 3);

wherein the subscript A refers to a component of the oil phase and B refers to the reactive building block having the highest molecular weight;

wherein the Hansen Solubility Parameters are calculated by the Yamamoto-Molecular Break (Y-MB) method implemented in HSPiP software Edition 4.

**3.** The method according to claim 1 and 2, wherein the oil phase comprising the benefit agent is split in a first part and a second part, wherein the first part fulfils the compatibility conditions a) to d) of claim 2, wherein the macromer is formed in this first part and wherein the second part of this oil phase is added once the macromer is formed.

**4.** The method according to any of the preceding claims, wherein the weight ratio of second to first parts is between 4.5 to 0.5 and 0.5 to 4.5, particularly between 4 to 1 and 1 to 4, more particularly between 3 to 2 and 2 to 3.

**5.** The method according to any of the preceding claims, wherein the macromer is an adduct formed by reaction of reactive building blocks consisting of an aminosilane and a polyfunctional isocyanate, and the polymeric stabilizer is formed by polycondensation of this adduct at the oil/water interface.

**6.** The method according to any of the preceding claims, wherein the hydrated polymer shell in step e) is a cross-linked coacervate, more particularly a cross-linked complex coacervate formed from a positively charged polyelectrolyte and a negatively charged polyelectrolyte.

**7.** A composition comprising at least one core shell microcapsules obtainable by the method according to any of claims 1 to 6.

**8.** The composition according to claim 7, wherein the shell of the core-shell microcapsules comprises material derived from a polymeric stabilizer that is formed by the a polycondensation of an adduct formed by reaction of bis(3-(triethoxysilyl)propyl)amine and 2-ethylpropane-1,2,3-triyl tris((3-(isocyanatomethyl)phenyl)carbamate).

**9.** The composition according to claim 8, wherein the first part of the oil phase comprises:

a) less than 5 wt.-%, more particularly less than 1 wt.-%, still more particularly less than 0.1 wt.-% of amines, aldehydes, enols and halogenated components;

b) 40 wt.-% or more, more particularly 60 wt.-% or more, still more particularly 75 wt.-% or more still more particularly 90 wt.-% or more, still more particularly 95 wt.-% or more of components having δD, δP, and δH fulfilling the conditions given by Equation 4 and referred to as GROUP 1 components:

$$D = \sqrt{4(\delta D - 19.077)^2 + (\delta P - 10.76)^2 + (\delta H - 4.81)^2} < 9$$

(Equation 4)

c) Less than 60 wt.-%, more particularly less than 40 wt.-%, still more particularly less than 25 wt.-%, still more particularly less than 10 wt.-%, still more particularly less than 5 wt.-% of components having Hansen solubility parameters δD, δP, and δH fulfilling the conditions given by Equation 5, and referred to as GROUP 2 components:

$$9 < D = \sqrt{4(\delta D - 19.077)^2 + (\delta P - 10.76)^2 + (\delta H - 4.81)^2} \leq 10$$

(Equation 5)

d) less than 1 wt.-% of components, having Hansen solubility parameters δD, δP, and δH fulfilling the conditions given by Equation 6:

$$D = \sqrt{4(\delta D - 19.077)^2 + (\delta P - 10.76)^2 + (\delta H - 4.81)^2} > 10$$

(Equation 6).

e) less than 40 wt.-% of components having a ClogP from 2.5 to 3.0 and less than 5 wt.-% of components having a ClogP of less than 2.5; and

f) less than 30 wt.-% of components having a molar volume of from 150 $cm^3$/mol to 170 $cm^3$/mol and less than 5 wt.-% of components having a molar volume of less than 150 $cm^3$/mol;

wherein the Hansen Solubility Parameters are calculated by the Yamamoto-Molecular Break (Y-MB) method implemented in HSPiP software Edition 4.

**10.** The composition according to claims 8 or 9, wherein the first oil phase GROUP 1 component is a fragrance ingredient selected from the group consisting of (2-(1-propoxyethoxy)ethyl)benzene, (Z)-oxacycloheptadec-10-en-2-one, pentyl 2-phenylacetate, 3,5-diethyl-2,5-dimethylcyclohex-2-enone, 7-isopentyl-2H-benzo[b][1,4]dioxepin-3(4H)-one, benzyl benzoate, benzyl 3-phenylprop-2-enoate, benzyl 2-methylpropanoate, benzyl 3-methylbutanoate, benzyl 2-phenylacetate, benzyl 2-hydroxybenzoate, 1-butoxy-1-oxopropan-2-yl butanoate, ((1S,8aR)-1,4,4-trimethyl-2,3,3a,4,5,8-hexahydro-1H-5,8a-methanoazulen-6-yl)methanol, (1S,6R,8aR)-1,4,4,6-tetramethyloctahydro-1H-5,8a-methanoazulen-6-ol, (4Z,8Z)-1,5,9-trimethyl-13-oxabicyclo[10.1.0]trideca-4,8-diene, methyl 2-(3-oxo-2-pentylcyclopentyl)acetate, 3-phenylprop-2-enyl 3-phenylprop-2-enoate, (E)-1,1-dimethoxy-3,7-dimethylocta-2,6-diene, (Z)-1,1-diethoxy-3,7-dimethylocta-2,6-diene, 3,7-dimethyloct-6-en-1-yl ethyl oxalate, 3,7-dimethyloct-6-en-1-yl formate, 3,7-dimethyloct-6-enenitrile, (Z)-3-methylcyclotetradec-5-enone, (4-methylphenyl) octanoate, (4-methylphenyl) 2-phenylacetate, 2-(4-methylphenoxy)acetaldehyde, allyl 2-(cyclohexyloxy)acetate, 2-cyclohexylethyl acetate, cyclohexyl 2-hydroxybenzoate, 3-(4-methylcyclohex-3-en-1-yl)butan-1-ol, (E)-1-(2,6,6-trimethylcyclohexa-1,3-dien-1-yl)but-2-en-1-one, (E)-1-(2,6,6-trimethylcyclohex-2-en-1-yl)but-2-en-1-one, (E)-1-(2,6,6-trimethyl-1-cyclohexenyl)but-2-en-1-one, 1-(2,6,6-trimethyl-1-cyclohex-3-enyl)but-2-en-1-one, decanenitrile, (oxybis(methylene))dibenzene, 3-methyl-2-pentylcyclopent-2-enone, 6-heptyltetrahydro-2H-pyran-2-one, 5-octyloxolan-2-one, 1,4-dioxacycloheptadecane-5,17-dione, 3-(4-ethylphenyl)-2,2-dimethylpropanenitrile, (E)-undec-9-enenitrile, (3aP,6S,7aS)-3a,4,5,6,7,7a-hexahydro-1H-4,7-methanoinden-6-yl propanoate, 2,4,6-trimethyl-4-phenyl-1,3-dioxane, (3aS,4S,7R,7aS)-ethyl octahydro-1H-4,7-methanoindene-3a-carboxylate, 2-methyldecanenitrile, 1-(1,2,8,8-tetramethyl-1,2,3,4,5,6,7,8-octahydronaphthalen-2-yl)ethanone, (E)-6,10-dimethylundeca-5,9-dien-2-one, (E)-oxacyclohexadec-12-en-2-one, methyl 3-oxo-2-pentylcyclopentaneacetate, heptan-2-one, (Z)-hex-3-en-1-yl benzoate, (Z)-hex-3-en-1-yl (Z)-hex-3-enoate, (Z)-hex-3-en-1-yl 2-hydroxybenzoate, (Z)-hex-3-enyl] (E)-2-methylbut-2-enoate, hexyl 2-hydroxybenzoate, (E)-4-(2,6,6-trimethylcyclohex-1-en-1-yl)but-3-en-2-one, (E)-3-methyl-4-(2,6,6-trimethylcyclohex-2-en-1-yl)but-3-en-2-one, (E)-4-(2,6,6-trimethylcyclohex-2-en-1-yl)but-3-en-2-one, 1-(2,3,8,8-tetramethyl-1,2,3,4,5,6,7,8-octahydronaphthalen-2-yl)ethanone, 2-methylpropyl 2-phenylacetate, 2-methylpropyl 2-hydroxybenzoate, 2-hexylcyclopent-2-en-1-one, 2-hexylcyclopent-2-enone, (E)-3-methyl-4-(2,6,6-trimethylcyclohex-2-en-1-yl)but-3-en-2-one, (3aR,6S,7aS)-3a,4,5,6,7,7a-hexahydro-1H-4,7-methanoinden-6-yl acetate, 2-hexylcyclopentanone, (E)-6-(pent-3-en-1-yl)tetrahydro-2H-pyran-2-one, 3-butyl-5-methyltetrahydro-2H-pyran-4-yl acetate, 3-pentyltetrahydro-2H-pyran-4-yl acetate, (4Z)-hept-4-en-2-yl 2-hydroxybenzoate, (2E,6Z)-3,7-dimethylnona-2,6-dienenitrile, 3-methyl-5-phenylpentan-1-ol, 5-methyl-2-propan-2-ylcyclohexyl] 2-hydroxypropanoate, methyl non-2-ynoate, (E)-3-methyl-4-(2,6,6-trimethylcyclohex-1-en-1-yl)but-3-en-2-one, (Z)-3-methylcyclopentadec-5-enone, 1,4-dioxacyclohexadecane-5,16-dione, 1,7-dioxacycloheptadecan-8-one, (4-(4-methylpent-3-en-1-yl)cyclohex-3-en-1-yl)methyl acetate, 2-(2-(4-methylcyclohex-3-en-1-yl)propyl)cyclopentan-1-one, (E)-methyl non-2-enoate, (E)-13-methyloxacyclopentadec-10-en-2-one, 4,4a-dimethyl-6-(prop-1-en-2-yl)-4,4a,5,6,7,8-hexahydronaphthalen-2(3H)-one, 4-(tert-pentyl)cyclohexanone, 2-ethyl-N-methyl-N-(m-tolyl)butanamide, 1,1-dimethoxynon-2-yne, 5-heptyldihydrofuran-2(3H)-one, 2-cyclohexylidene-2-phenylacetonitrile, 3,7-dimethylocta-1,6-dien-3-yl dimethylcarbamate, 2-cyclohexylidene-2-(o-tolyl)acetonitrile, 2-(phenoxy)ethyl 2-methylpropanoate, 2-phenylethyl 3-phenylprop-2-enoate, 2-phenylethyl 2-methylpropanoate, 2-phenylethyl 2-phenylacetate, 2-phenylethyl 2-hydroxybenzoate, (2E,5E)-5,6,7-trimethylocta-2,5-dien-4-one, (4aR,8aS,E)-6-ethylideneoctahydro-2H-5,8-methanochromene, 3-(2-methylpropyl)-1-methylcyclohexanol, 2,3,3-trimethyl-1-indanone, 4,5,6,7,8,9,10,11,12,13-decahydrocyclododeca[d]-oxazole, 1-(spiro[4.5]dec-6-en-7-yl)pent-4-en-1-one, (E)-tridec-2-enenitrile, 6-hexyltetrahydro-2H-pyran-2-one, (Z)-cyclohexadec-5-enone, or mixtures thereof; and, wherein the first oil phase GROUP 2 component is a fragrance ingredient selected from the groups consisting of octyl acetate, (Z)-4,11,11-trimethyl-8-methylenebicyclo[7.2.0]undec-4-ene, 2-methyl-4-(5,6,6-trimethylbicyclo[2.2.1]hept-2-yl-cyclohexanone, prop-2-enyl 2-(3-methylbutoxy)acetate, prop-2-enyl heptanoate, 1-((2-(tert-butyl)cyclohexyl)oxy)butan-2-ol, 1,3,4,5,6,7-hexahydro-.beta.,1,1,5,5-pentamethyl-2H-2,4a-Methanonaphthalene-8-ethanol, (2-(isopentyloxy)ethyl)benzene, 1-(3,3-dimethylcyclohexyl)ethyl formate, (E)-2-ethyl-4-(2,2,3-trimethylcyclopent-3-en-1-yl)but-2-en-1-ol, (1R,2S,4R)-2'-isopropyl-1,7,7-trimethylspiro[bicyclo[2.2.1]heptane-2,4'-[1,3]dioxane], 2-methyl-6-methyleneoct-7-en-2-yl acetate, (ethoxymethoxy)cyclododecane, (2S,4S)-1,7,7-trimethylbicyclo[2.2.1]heptan-2-yl acetate, 4-(tert-butyl)cyclohexanol, 1,1,2,3,3-pentamethyl-2,3,6,7-tetrahydro-1H-inden-4(5H)-one, ((3R,3aS,7R,8aS)-3,8,8-trimethyl-2,3,4,7,8,8a-hexahydro-1H-3a,7-methanoazulen-6-yl)methyl acetate, (1S,6R,8aR)-1,4,4,6-tetramethyloctahydro-1H-5,8a-methanoazulen-6-yl acetate, 2-methyl-4-(2,6,6-trimethylcyclohex-2-en-1-yl)butanal, 3,7-dimethyloct-6-en-1-ol, 3,7-dimethyloct-6-en-1-ol, 3,7-dimethyloct-6-en-1-yl acetate, 3,7-dimethyloct-6-en-1-yl propanoate, 4-cyclohexyl-2-methylbutan-2-ol, ethyl 2,6,6-trimethylcyclohexa-1,3-diene-1-carboxylate, 3,7-dimethyloct-6-en-3-ol, 2,6-dimethyloct-7-en-2-ol, 2-methyl-1-phenylpropan-2-yl butanoate, (E)-3-methyl-5-(2,2,3-trimethylcyclopent-3-en-1-yl)pent-4-en-2-ol, (2E,4Z)-ethyl deca-2,4-dienoate, ethyl decanoate, (E)-3,7-dimethylnona-1,6-dien-3-ol, ethyl octanoate, ethyl nonanoate, ethyl 2,6,6-trimethylcyclohexa-1,3-diene-1-carboxylate, (2S)-1,3,3-trimethylbicyclo[2.2.1]heptan-2-yl acetate, 1-(3,5,5,6,8,8-hexamethyl-5,6,7,8-tetrahydronaphthalen-2-yl)ethanone, 2,6-dimethyloct-7-en-2-ol, (Z)-1-(cyclooct-3-en-1-yl)propan-1-ol, 4,4,8,8-tetramethyloctahydro-4a,7-methanonaphtho[1,8a-b]oxirene, (3aR,6S,7aS)-3a,4,5,6,7,7a-hexahydro-1H-4,7-methanoinden-6-yl 2-methyl propanoate, (E)-3,7-dimethylocta-2,6-dien-1-yl acetate, 2-(8-isopropyl-6-methylbicyclo[2.2.2]oct-5-en-2-yl)-1,3-dioxolane, (Z)-hex-3-en-1-yl butanoate, (Z)-hex-3-en-1-yl 2-methylpropanoate, (Z)-hex-3-en-1-yl 2-methyl butanoate, hexyl butanoate, hexyl 2-methylpropanoate, (E)-4-(2,5,6,6-tetramethyl-1-cyclohex-2-enyl)but-3-en-2-one, (E)-4-(2,5,6,6-tetramethylcyclohex-2-en-1-yl)but-3-en-2-one, (E)-4-(2,5,6,6-tetramethylcyclohex-2-en-1-yl)but-3-en-2-one, 2,2,7,7-tetramethyltricyclo[6.2.1.01,6]undecan-5-one, (1-methyl-2-((1,2,2-trimethylbicyclo[3.1.0]hexan-3-yl)methyl)cyclopropyl)methanol, 1-(4-methoxy-2,2,6,6-tetramethylcyclohex-3-en-1-yl)ethanone, (Z)-1-(1-ethoxyethoxy)hex-3-ene, 3,7-dimethylocta-1,6-dien-3-ol, 3,7-dimethylocta-1,6-dien-3-yl formate, 2-(4-methylcyclohexyl)propan-2-yl acetate, 2-isopropyl-5-methylcyclohexanol, 1-((1S,8aS)-1,4,4,6-tetramethyl-2,3,3a,4,5,8-hexahydro-1H-5,8a-methanoazulen-7-yl)-ethanone, (2-methyl-6-methylideneoct-7-en-2-yl) acetate, 2-methylundecanoic acid, 2-methyl-6-methyleneoct-7-en-2-yl acetate, 10-isopropyl-2,7-dimethyl-1-oxaspiro[4.5]deca-3,6-diene, (E)-3,7,11-trimethyldodeca-1,6,10-trien-3-ol, (Z)-3,7-dimethylocta-2,6-dien-1-yl acetate, 2-(6,6-dimethylbicyclo[3.1.1]hept-2-en-2-yl)ethyl acetate, 2,4-dimethyl-2-(5,5,8,8-tetramethyl-5,6,7,8-tetrahydronaphthalen-2-yl)-1,3-dioxolane, (1-methyl-2-(((1R,3R)-2,2,3-trimethylcyclopentyl)methyl)-cyclopropyl)methanol, 2,2-dimethyl-2-pheylethyl propanoate, ethyl 2-cyclohexylpropanoate, (E)-2-ethyl-4-(2,2,3-trimethylcyclopent-3-en-1-yl)but-2-en-1-ol, 2-(cyclohexylmethyl)-4,4,6-trimethyl-1,3-dioxane, mixture of 3,7-dimethyloct-6-en-1-ol and 3,7-dimethyloct-7-en-1-ol, dec-9-en-1-ol, 3-((1R,2S,4R,6R)-5,5,6-trimethylbicyclo[2.2.1]heptan-2-yl)cyclohexanol, 3-((1R,2S,4R,6R)-5,5,6-trimethylbicyclo[2.2.1]heptan-2-yl)cyclohexanol, (E)-2-methyl-4-(2,2,3-trimethyl-1-cyclopent-3-enyl)but-2-en-1-ol, 2-(1-(3,3-dimethylcyclohexyl)ethoxy)-2-methylpropyl cyclopropanecarboxylate, (E)-6-ethyl-3-methyloct-6-en-1-ol, (E)-2-((3,5-dimethylhex-3-en-2-yl)oxy)-2-methylpropyl cyclopropanecarboxylate, (E)-6,10-dimethylundeca-5,9-dien-2-yl acetate, 2-(4-methyl-1-cyclohex-3-enyl)propan-2-yl acetate, oxacyclohexadecan-2-one, (E)-4-methyldec-3-en-5-ol, 2,2,5-trimethyl-5-pentylcyclopentanone, 1-((1S,8aS)-1,4,4,6-tetramethyl-2,3,3a,4,5,8-hexahydro-1H-5,8a-methanoazulen-7-yl)ethanone, 4-methyl-4-phenylpentan-2-yl acetate, (4,8-dimethyl-2-propan-2-ylidene-3,3a,4,5,6,8a-hexahydro-1H-azulen-6-yl) acetate, (2R,5R,8S)-4,4,8-trimethyltricyclo[6.3.1.02,5]dodecan-1-yl acetate, [(3Z)-4,11,11-trimethyl-8-methylidene-5-bicyclo[7.2.0]undec-3-enyl] acetate, or mixtures thereof.

**11.** The composition according to any of claim 8 to 10, wherein the second oil phase comprises, based on the total weight of the second oil phase:

a) Less than 5 wt.-%, more particularly less than 1 wt.-%, still more particularly less than 0.1 wt.-% of amines

and alpha-beta unsaturated aldehydes;

b) 15 wt.-% or less of aldehydes that are not alpha-beta unsaturated aldehydes;

c) Less than 40 wt.-% of components having a ClogP from 2.5 to 3.0 and less than 5 wt.-% of components having a ClogP of less than 2.5;

d) Less than 30 wt.-% of components having a molar volume of from 150 $cm^3$/mol to 170 $cm^3$/mol and less than 5 wt.-% of components having a molar volume of less than 150 $cm^3$/mol;

e) 50 wt.-% or less of components having Yamamoto-Molecular Break (Y-MB) Hansen solubility parameters $\delta D$, $\delta P$, and $\delta H$ fulfilling the conditions given by Equation 7:

$$\sqrt{4(\delta D - 19.077)^2 + (\delta P - 10.76)^2 + (\delta H - 4.81)^2} > 10$$

(Equation 7).

**12.** The composition according to any of claims 8 to 11, wherein the complex coacervate comprises:

a) a positively charged polyelectrolyte selected from gelatin, chitosan and permanently charged cationic polysaccharide selected from cationic hydroxypropyltrimonium starch or hydroxypropyltrimonium guar gum, and mixtures thereof; and

b) a negatively charged polyelectrolyte selected from the group consisting of pectin, gum Arabic and alginate, and their sodium, potassium, magnesium or calcium carboxylate salts, and mixtures thereof, preferably pectin and its salts.

**13.** The composition according to any of claims 8 to 12, wherein the coacervate to polymeric stabilizer weight ratio is from 1 to 3, preferably from 1.5 to 2.5.

**14.** Use of an encapsulated composition according to any of claims 8 to 13 to enhance the performance of a benefit agent in a consumer product.

**15.** A consumer product comprising an encapsulated composition according to any of claims 8 to 13, preferably wherein the consumer product is selected from the group consisting of fabric care detergents and conditioners, hair care conditioners, shampoos, heavy duty liquid detergents, hard surface cleaners, detergent powders, soaps, shower gels and skin care products.

Europäisches Patentamt
European Patent Office
Office européen des brevets

# EUROPEAN SEARCH REPORT

Application Number
EP 23 16 7736

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2023/020883 A1 (GIVAUDAN SA [CH]) 23 February 2023 (2023-02-23) * Description page 31, lines 8-16.; example 1 * ----- | 1-15 | INV. B01J13/10 |
| X | WO 2022/023110 A1 (GIVAUDAN SA [CH]) 3 February 2022 (2022-02-03) * page 28, line 25 - 32; page 29, lines 28-32); example 7 * ----- | 1-15 | |
| A | WO 2022/112202 A1 (GIVAUDAN SA [CH]) 2 June 2022 (2022-06-02) * example 11 * ----- | 1-15 | |
| A | WO 2021/239742 A1 (GIVAUDAN SA [CH]) 2 December 2021 (2021-12-02) * example 1 * ----- | 1-15 | |
| A | US 2022/280398 A1 (HABAR GÉRARD [FR]) 8 September 2022 (2022-09-08) * claim 1; example 1 * ----- | 1-15 | TECHNICAL FIELDS SEARCHED (IPC) |
| A | US 2022/175635 A1 (VAN GRUIJTHUIJSEN KITTY [CH] ET AL) 9 June 2022 (2022-06-09) * protocl 5, ing [0425] * ----- | 1-15 | B01J |

The present search report has been drawn up for all claims

1

| Place of search | Date of completion of the search | Examiner |
|---|---|---|
| The Hague | 21 September 2023 | Mc Donnell, Shane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 16 7736

This annex lists the patent family members relating to the patent documents cited in the above-mentioned European search report. The members are as contained in the European Patent Office EDP file on
The European Patent Office is in no way liable for these particulars which are merely given for the purpose of information.

21-09-2023

| Patent document cited in search report | Publication date | Patent family member(s) | Publication date |
|---|---|---|---|
| WO 2023020883 A1 | 23-02-2023 | NONE | |
| WO 2022023110 A1 | 03-02-2022 | BR 112023001542 A2 | 11-04-2023 |
| | | CN 115942990 A | 07-04-2023 |
| | | EP 4188589 A1 | 07-06-2023 |
| | | JP 2023536823 A | 30-08-2023 |
| | | KR 20230042354 A | 28-03-2023 |
| | | US 2023212483 A1 | 06-07-2023 |
| | | WO 2022023110 A1 | 03-02-2022 |
| WO 2022112202 A1 | 02-06-2022 | CN 116507709 A | 28-07-2023 |
| | | EP 4251723 A1 | 04-10-2023 |
| | | KR 20230107680 A | 17-07-2023 |
| | | WO 2022112202 A1 | 02-06-2022 |
| WO 2021239742 A1 | 02-12-2021 | BR 112022022587 A2 | 13-12-2022 |
| | | CN 115666494 A | 31-01-2023 |
| | | EP 4157203 A1 | 05-04-2023 |
| | | JP 2023527825 A | 30-06-2023 |
| | | US 2023166230 A1 | 01-06-2023 |
| | | WO 2021239742 A1 | 02-12-2021 |
| US 2022280398 A1 | 08-09-2022 | BR 112022001462 A2 | 07-06-2022 |
| | | CN 114206488 A | 18-03-2022 |
| | | EP 4010110 A1 | 15-06-2022 |
| | | FR 3099711 A1 | 12-02-2021 |
| | | US 2022280398 A1 | 08-09-2022 |
| | | WO 2021023922 A1 | 11-02-2021 |
| US 2022175635 A1 | 09-06-2022 | BR 112021018269 A2 | 08-02-2022 |
| | | CN 113557082 A | 26-10-2021 |
| | | EP 3921074 A1 | 15-12-2021 |
| | | JP 2022542633 A | 06-10-2022 |
| | | SG 11202109580Q A | 28-10-2021 |
| | | US 2022175635 A1 | 09-06-2022 |
| | | WO 2021018947 A1 | 04-02-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020233887 A1
- WO 2023020883 A1
- GB 2203193 A